# FASCICULE DE BREVET EUROPEEN

(11) **EP 1 910 376 B1**
(45) Date de publication et mention de la délivrance du brevet: **30.09.2009**
(21) Numéro de dépôt: 06794287.0
(22) Date de dépôt: 04.08.2006
(51) Int. Cl.: C07D 491/22, C07D 471/14, A61K 31/4745

(54) **NOUVEAUX COMPOSES ANALOGUES DE LA CAMPTOTHECINE, LEUR PROCEDE DE PREPARATION ET LES COMPOSITIONS PHARMACEUTIQUES QUI LES CONTIENNENT**
NEUE CAMPTOTHECIN-ANALOGA-VERBINDUNGEN, HERSTELLUNGSVERFAHREN DAFÜR UND DIESE ENTHALTENDE PHARMAZEUTISCHE ZUSAMMENSETZUNGEN
NOVEL CAMPTOTHECIN ANALOGUES COMPOUNDS, A METHOD FOR THE PREPARATION THEREOF AND PHARMACEUTICAL COMPOSITIONS CONTAINING SAID COMPOUNDS

(30) Priorité: 05.08.2005 FR 0508365
(43) Date de publication de la demande: 16.04.2008
(73) Titulaire: Les Laboratoires Servier, 92415 Courbevoie Cedex (FR)
(72) Inventeur: LAVIELLE, Gilbert, F-78170 La Celle Saint Cloud (FR); HAUTEFAYE, Patrick, F-77170 Servon Brie Comte Robert (FR); PIERRE, Alain, F-78580 Les Alluets le Roi (FR); HICKMAN, John, F-75017 Paris (FR); LEONCE, Stéphane, F-78000 Versailles (FR)
(86) Numéro de dépôt international: PCT/FR2006/001900
(87) Numéro de publication internationale: WO 2007/017584

(56) Documents cités:
- EP-A- 1 101 765
- US-A1- 2003 105 109

## Description

La présente invention concerne de nouveaux composés analogues de la camptothécine, possédant un cycle E cétonique à substituant aminoalkylcarbonyloxy ou dérivé dudit substituant, leur procédé de préparation et les compositions pharmaceutiques qui les contiennent.

La camptothécine (CPT), alcaloïde isolé de *Camptothéca accuminata,* est un agent anticancéreux possédant un large spectre d'activité. Son caractère insoluble a longtemps orienté les recherches vers les sels solubles qui se sont avérés inactifs et toxiques.

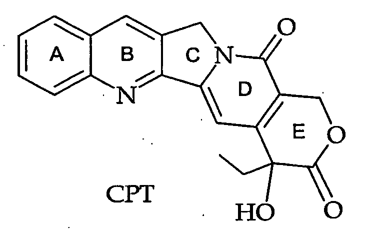

Un autre problème provient du manque de stabilité du cycle E. En effet, la fonction lactone du cycle E, est en équilibre dans les milieux physiologiques avec sa forme ouverte hydroxy-acide. Cette dernière est inactive et semble avoir une toxicité particulière intrinsèque [Cancer Research., 49, 1465 (1989); ibid, 49, 5077 (1989)]. Des tentatives de modification de ce cycle, afin de le rendre plus stable, ont été menées, en particulier l'atome d'oxygène cyclique a été remplacé par un atome d'azote ou de soufre, mais dans chaque cas il y a perte de l'activité pharmacologique confirmant ainsi l'importance de la lactone *[*Journal of Medicinal Chemistry, 32, 715 (1989)]. D'autres modifications structurales du cycle E de la CPT ont été décrites par la suite, en particulier dans le brevet EP 1101765. Ces nouveaux composés se caractérisent par le remplacement de la lactone par une fonction cétonique cyclique.

La présente invention concerne des analogues de la camptothécine comportant une fonction cétonique sur le cycle E à cinq chaînons et possédant sur ce même cycle un groupement aminoalkylcarbonyloxy, ou un de ses dérivés, qui substitue la fonction hydroxyle en alpha de la cétone.

Cette modification apporte aux composés de l'invention une activité pharmacologique exacerbée notamment dans leur caractère cytotoxique.

Ils pourront donc être utilisés pour la fabrication de médicaments utiles dans le traitement des maladies cancéreuses.

L'invention concerne les composés de formule (I) : 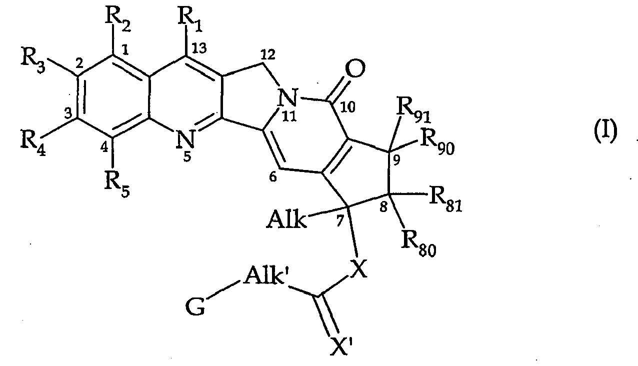 dans laquelle :
- Alk représente un groupement alkyle,
- R₁, R₂, R₃, R₄ et R₅ sont choisis indépendamment parmi un atome d'hydrogène, d'halogène, un groupement alkyle, alkényle, alkynyle, polyhalogénoalkyle, cycloalkyle éventuellement substitué, cycloalkylalkyle éventuellement substitué, aryle éventuellement substitué, hydroxy, hydroxyalkyle, alkoxy, alkoxyalkyle, nitro, cyano, acyloxy, -C(O)-R, et les groupements -(CH₂)p-NRₐR_{b}, et -O-C(O)-N-RₐR_{b}, dans lesquels R représente un groupement alkyle, alkoxy ou amino(éventuellement substitué sur l'atome d'azote par un ou deux groupements alkyle), p est un entier compris entre 0 et 6, et Rₐ et R_{b} représentent indépendamment un atome d'hydrogène, un groupement alkyle, cycloalkyle, cycloalkylalkyle, acyle, aryle éventuellement substitué, arylalkyle éventuellement substitué, ou bien Rₐ et R_{b} forment ensemble avec l'atome d'azote qui les porte un groupement pyrrolyle, pipéridinyle, ou pipérazinyle, chacun de ces groupements cycliques pouvant être éventuellement substitué,
   ou bien deux groupements R₂, R₃, R₄ et R₅ adjacents forment ensemble avec les atomes de carbone qui les portent un groupement -T-(CR_{c}R_{d})ₜ-T'-, dans lequel T et T', identiques ou différents, représentent un atome d'oxygène, de souffre ou un groupement N-Rₑ ; R_{c} et R_{d}, identiques ou différents, représentent un atome d'hydrogène ou d'halogène ; t est un entier compris entre 1 et 3 inclus ; et Re représente un atome d'hydrogène, un groupement alkyle ou benzyle,
- R₈₀ et R₉₀ représentent indépendamment un atome d'hydrogène, un groupement hydroxy, alkyle, ou alkoxy,
- R₈₁ et R₉₁ représentent indépendamment un atome d'hydrogène, un groupement alkyle, alkényle, alkynyle, ou, pris deux à deux sur des carbones adjacents, forment ensemble une liaison, ou un groupement oxirane, ou bien deux groupements géminaux (R₈₀ et R₈₁) et/ou (R₉₀ et R₉₁) forment ensemble un groupement oxo ou un groupement -O-(CH₂)ₜ₁-O-, t₁ étant un entier compris entre 1 et 3 inclus,
- X et X', identiques ou différents, représentent un atome d'oxygène, de soufre, un groupement amino ou alkylamino,
- Alk' représente une chaîne alkylène, alkénylène, ou alkynylène,
- G représente un groupement NR₆R₇ où:
   i) soit R₆, R₇ représentent indépendamment l'un de l'autre un atome d'hydrogène, un groupement alkyle, cycloalkyle, aryle éventuellement substitué, arylalkyle éventuellement substitué, cycloalkyle éventuellement substitué, cycloalkylalkyle éventuellement substitué, hétéroaryle éventuellement substitué, ou hétéroarylalkyle éventuellement substitué,
   ii) soit R₆ et R₇ forment ensemble avec l'atome d'azote un groupement hétérocycloalkyle monocyclique 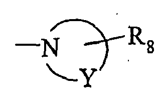 de 5 à 8 chaînons ou bicyclique 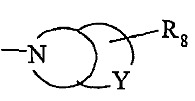 de 5 à 11 chaînons dons lesquels :
      ■ Y représente un atome d'azote, d'oxygène ou un groupement CH₂ et
      ■ R₈ représente un atome d'hydrogène, un groupement alkyle, cycloalkyle éventuellement substitué, cycloalkylalkyle éventuellement substitué, aryle éventuellement substitué, arylalkyle éventuellement substitué, hétérocycloalkyle éventuellement substitué, hétérocycloalkylalkyle éventuellement substitué, hétéroaryle éventuellement substitué, hétéroarylalkyle éventuellement substitué,
leurs énantiomères, diastéréoisomères, ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- le terme alkényle désigne une chaîne de 2 à 6 atomes de carbone, linéaire ou ramifié et contenant de 1 à 3 doubles liaisons,
- le terme alkynyle désigne une chaîne de 2 à 6 atomes de carbone, linaire ou ramifié et contenant de 1 à 3 triples liaisons,
- le terme alkylène désigne un radical bivalent, linéaire ou ramifié, contenant de 1 à 6 atomes de carbone,
- le terme alkénylène désigne un radical bivalent, linéaire ou ramifié, contenant de 2 à 6 atomes de carbone et de 1 à 3 doubles liaisons,
- le terme alkynylène désigne un radical bivalent linéaire ou ramifié, contenant de 2 à 6 atomes de carbones et de 1 à 3 triples liaisons,
- le terme acyle désigne un radical alkyle-carbonyle linéaire ou ramifié contenant de 1 à 6 atomes de carbone,
- le terme alkoxy désigne un radical alkyle-oxy dont le groupement alkyle est linéaire ou ramifié, contenant de 1 à 6 atomes de carbone,
- le terme acyloxy désigne un radical acyle-oxy dont le groupement acyle est un radical akylcarbonyle linéaire ou ramifié,
- le terme aryloxyalkyle désigne un groupement aryle-oxy-alkyle dont le groupement alkyle est linéaire ou ramifié, contenant de 1 à 6 atomes de carbone,
- les termes arylalkyl, cycloalylalkyle, hétéroarylalkyle, hétérocycloalkylalkyle désignent les radicaux aryle-alkyle, cycloalkyle-alkyle, hétéroaryle-alkyle, hétérocycloalkyle-alkyle dans lesquels le groupement alkyle désigne une chaîne de 1 à 6 atomes de carbone, linéaire ou ramifié,
- le terme polyhalogénoalkyle désigne une chaîne carbonée, linéaire ou ramifiée, contenant de 1 à 3 atomes de carbone et de 1 à 7 atomes d'halogène,
- le terme halogène désigne les atomes de fluor, de chlore, de brome ou d'iode,
- le terme aryle désigne un groupement phényle, naphtyle, indanyle, indényle, dihydronaphtyle ou tétrahydronaphtyle,
- le terme cycloalkyle désigne un monocycle ou bicycle hydrocarboné comportant de 3 à 11 atomes de carbone, et éventuellement insaturé par 1 ou 2 insaturations,
- le terme hétéroaryle désigne un groupement monocyclique ou bicyclique dans lequel au moins un des cycles est aromatique, comportant de 5 à 11 chaînons et de 1 à 4 hétéroatomes, choisis parmi l'azote, l'oxygène et le soufre,
- le terme hétérocycloalkyle désigne un groupement mono ou bicyclique, saturé ou insaturé par 1 ou 2 insaturations, contenant de 4 à 11 chaînons et possédant de 1 à 4 hétéroatomes choisis parmi azote, oxygène et soufre,
- l'expression "éventuellement substitué" lorsqu'elle concerne les groupements aryle ou arylalkyle ; cycloalkyle ou cycloalkylalkyle ; hétéroaryle ou hétéroarylalkyle ; et hétérocycloalkyle ou hétérocycloalkylalkyle ; signifie que les groupements aryle ; cycloalkyle ; hétéroaryle ; et hétérocycloalkyle respectivement peuvent être substitués par 1 à 3 substituants, identiques ou différents, choisis parmi l'atome d'halogène, groupement alkyle, alkoxy, alkylthio, alkylsulfinyle, alkylsulfonyle, hydroxy, mercapto, cyano, nitro, amino (éventuellement substitué par un ou deux groupements alkyle), acyle, formyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy et sulfo,
- l'expression "éventuellement substitué" lorsqu'elle concerne les groupements pyrrolyle, pipéridinyle, ou pipérazinyle signifie que les groupements concernés peuvent être substitués par 1 à 3 groupements, identiques ou différents, choisis parmi alkyle, alkoxy, aryle, arylalkyle, aryloxy, et aryloxyalkyle.

Un aspect avantageux de l'invention concerne les composés de formule (I) pour lesquels Alk représente un groupement éthyle.

Un autre aspect avantageux de l'invention concerne les composés de formule (I) pour lesquels R₈₀ et R₈₁ forment ensemble un groupement oxo, ou bien R₉₀ et R₉₁ forment ensemble un groupement oxo, ou bien R₈₀ et R₈₁ ainsi que R₉₀ et R₉₁ forment deux groupements oxo. Plus avantageusement R₈₀ et R₈₁ forment ensemble un groupement oxo, et R₉₀ et R₉₁ représentent chacun un atome d'hydrogène.

Des composés préférés de formule (I) sont ceux pour lesquels R₅ représente un atome d'hydrogène.

D'autres composés préférés de formule (I) sont ceux pour lesquels R₂, R₃ et R₄ sont choisis parmi un atome d'hydrogène, d'halogène, un groupement alkyle ou alkoxy.

D'autres composés préférés de formule (I) sont ceux pour lesquels R₃ et R₄ forment ensemble un groupement méthylènedioxy ou éthylènedioxy (préférentiellement méthylènedioxy).

Des composés avantageux de formule (I) sont ceux pour lesquels R₂ représente un atome d'hydrogène.

Un aspect particulièrement avantageux de l'invention concerne les composés de formule (I) pour lesquels R₁ représente un groupement alkyle, cycloalkyle ou cycloalkylalkyle (préférentiellement cycloalkyle).

Un autre aspect avantageux de l'invention concerne les composés de formule (I) pour lesquels R₁ représente un groupement aryle éventuellement substitué (préférentiellement phényle).

Un autre aspect également avantageux de l'invention concerne les composés de formule (I) pour lesquels G représente un groupement NR₆R₇ où R₆ et R₇ forment ensemble avec l'atome d'azote un groupement hétérocycloalkyle monocyclique (avantageusement saturé) : 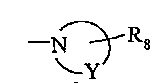 de 5 à 8 chaînons (plus avantageusement à 6 chaînons) dans lequel Y représente un atome d'azote, d'oxygène ou un groupement CH₂ (plus avantageusement CH₂) et R₈ représente un atome d'hydrogène ou un groupement alkyle (plus avantageusement hydrogène).

D'autres composés préférés sont ceux appartenant à la formule générale (I) pour lesquels Alk' représente un groupement alkylène (plus avantageusement -CH₂-CH₂-).

Des composés préférés de l'invention sont ceux pour lesquels X et X', identiques ou différents, représentent un atome d'oxygène ou de soufre (plus avantageusement d'oxygène).

Les composés particulièrement intéressant de l'invention sont le 3-piperidinopropanoate de 7-éthyl-2,3-méthylènedioxy-13-méthyl-8,10-dioxo-8,9,10,12-tétrahydro-7*H-*cyclopenta-[6,7]indolizino[1,2-*b*]quinolin-7-yl ; le 3-piperidinopropanoate de 7-éthyl-2,3-méthylènedioxy-13-cyclobutyl-8,10-dioxo-8,9,10,12-tétrahydro-7*H-*cyclopenta[6,7]-indolizino[1,2-*b*]quinolin-7-yl ; et le 3-hexahydrocyclopenta[*c*]pyrrol-2(1*H*)-ylpropanoate de 7-éthyl-2,3-méthylènedioxy-13-cyclobutyl-8,10-dioxo-8,9,10,12-tétrahydro-7*H-*cyclopenta[6,7]-indolizino[1,2-*b*]quinolin-7-yl.

La présente invention concerne également le procédé de préparation des composés de formule (I), caractérisé en ce que l'on utilise comme produit de départ un composé de formule (II) synthétisé comme décrit dans EP 1101765: 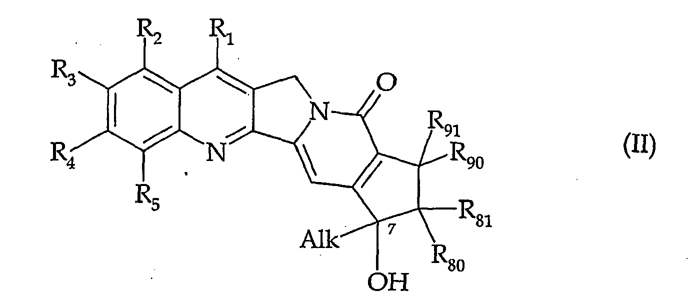 dans laquelle Alk, R₁, R₂, R₃, R₄, R₅ R₈₀, R₈₁, R₉₀ et R₉₁ sont tels que définis dans la formule (I),
dont le groupement hydroxy en C₇ est transformé en X"H avec X" représentant un groupement SH, amino, ou alkylamino pour conduire au composé de formule (III) 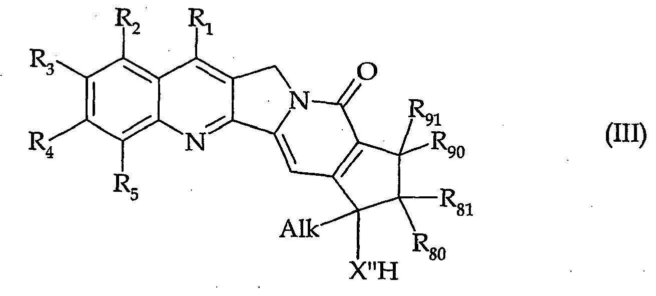 dans laquelle Alk, R₁, R₂, R₃, R₄, R₅ R₈₀, R₈₁, R₉₀ et R₉₁ sont tels que définis dans la formule (I), et X" est tel que défini précédemment,
composés de formule (II) ou (III) qui se condensent avec le réactif (IV) : 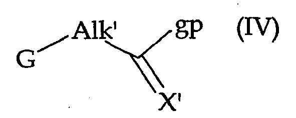 dans laquelle G, Alk' et X' sont tels que définis dans la formule (I), et gp un groupe partant tel que Hal, OH, SH, NR'R", OC(O)R' où R', R" représentent des groupements alkyles,
pour conduire au composé de formule (I),
étant entendu, dans le but de simplifier le procédé ci-dessus, que les groupements réactifs présents en R₈₀, R₈₁, R₉₀ et R₉₁ peuvent être protégés à l'aide de groupements protecteurs classiques et déprotégés au moment opportun, que les groupements hydroxy présents en ces mêmes positions peuvent être oxydés par des méthodes classiques de la chimie, en groupement oxo, qu'inversement les groupements oxo présents en ces mêmes positions peuvent être réduits par des réducteurs classiques, à tout moment opportun de la synthèse, et que lorsque deux de ces groupements forment ensemble une liaison, cette dernière peut être introduite à tout moment jugé utile par l'homme de métier afin de faciliter la synthèse,
composés de formule (I) :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

La présente invention concerne également les intermédiaires de synthèse (III') : 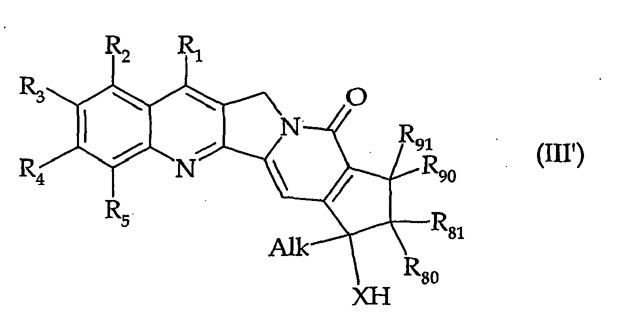 dans laquelle :
- Alk représente un groupement alkyle,
- R₁, R₂, R₃, R₄ et R₅ sont choisis indépendamment parmi un atome d'hydrogène, d'halogène, un groupement alkyle, alkényle, alkynyle, polyhalogénoalkyle, cycloalkyle éventuellement substitué, cycloalkylalkyle éventuellement substitué, hydroxy, hydroxyalkyle, alkoxy, alkoxyalkyle, nitro, cyano, acyloxy, -C(O)-R, et les groupements -(CH₂)p-NRₐR_{b}, et -O-C(O)-N-RₐR_{b}, dans lesquels R représente un groupement alkyle, alkoxy ou amino(éventuellement substitué sur l'atome d'azote par un ou deux groupements alkyle), p est un entier compris entre 0 et 6, et Rₐ et R_{b} représentent indépendamment un atome d'hydrogène, un groupement alkyle, cycloalkyle, cycloalkylalkyle, acyle, aryle éventuellement substitué, arylalkyle éventuellement substitué, ou bien Rₐ et R_{b} forment ensemble avec l'atome d'azote qui les porte un groupement pyrrolyle, pipéridinyle, ou pipérazinyle, chacun de ces groupements cycliques pouvant être éventuellement substitué, et au moins deux groupements R₂, R₃, R₄ et R₅ adjacents forment ensemble avec les atomes de carbone qui les portent un groupement -T-(CRcRd)t-T'-, dans lequel T et T', identiques ou différents, représentent un atome d'oxygène, de souffre ou un groupement N-Rₑ ; R_{c} et R_{d}, identiques ou différents, représentent un atome d'hydrogène ou d'halogène ; t est un entier compris entre 1 et 3 inclus ; et Re représente un atome d'hydrogène, un groupement alkyle ou benzyle, étant entendu qu'au moins un des deux groupements R_{c} ou R_{d} représente un atome d'halogène lorsque T et T' représentent chacun un atome d'oxygène et X représente un atome d'oxygène,
- R₈₀ et R₉₀ représentent indépendamment un atome d'hydrogène, un groupement hydroxy, alkyle, ou alkoxy,
- R₈₁ et R₉₁ représentent indépendamment un atome d'hydrogène, un groupement alkyle, alkényle, alkynyle, ou, pris deux à deux sur des carbones adjacents, forment ensemble une liaison, ou un groupement oxirane, ou bien deux groupements géminaux (R₈₀ et R₈₁) et/ou (R₉₀ et R₉₁) forment ensemble un groupement oxo ou un groupement -O-(CH₂)ₜ₁-O-, t₁ étant un entier compris entre 1 et 3 inclus,
- X représentent un atome d'oxygène, de souffre, un groupement amino ou alkylamino,
leurs énantiomères, diastéréoisomères, ainsi que leurs sels d'addition à un acide ou à une base pharmaceutiquement acceptable.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale (notamment intraveineuse). D'une manière générale, la posologie unitaire s'échelonne entre 0,1 et 500 mg pour un traitement en 1 à 3 prises par 24 heures.

Les exemples suivants illustrent l'invention et ne la limitent en aucune façon.

Les structures des composés décrits dans les exemples et les préparations ont été déterminées selon les techniques spectroprotométriques usuelles (infrarouge, RMN, spectrométrie de massue, ...).

Les composés de départ de formule (II), et (III') pour lesquels X représente un atome d'oxygène, ont été synthétisés selon les conditions expérimentales décrites dans le brevet EP 1101765 et adaptées à l'aide des documents de l'état de l'art, connu par l'homme du métier, aux composés de l'invention. A titre d'exemple, les préparations 1 à 6 viennent illustrer, et ne limitent en aucune façon, la manière d'adapter la synthèse décrite dans le brevet EP 1101765 aux composés de l'invention.

### PRÉPARATION 1 : 7-Ethyl-7-hydroxy-2,3-méthylènedioxy-13-méthyl-9,12-dihydro-7H-cyclopenta[6,7]indolizino[1,2-b]quinoline-8,10-dione

Le composé du titre est préparé selon la méthode décrite dans l'Exemple 11 du brevet EP 1101765, en remplaçant la 2-bromo-3-bromométhyl-6,7-méthylènedioxyquinoléine par la 2-bromo-3-bromométhyl-4-méthyl-6,7-méthylènedioxyquinoléine.

### PREPARATION 2 : 7-Ethyl-7-hydroxy-2,3-méthylènedioxy-13-cyclobutyl-9,12-dihydro-7H-cyclopenta[6,7]indolizino[1,2-b]quinoline-8,10-dione

Le composé du titre est préparé selon la méthode décrite dans l'Exemple 11 du brevet EP 1101765, en remplaçant la 2-bromo-3-bromométhyl-6,7-méthylènedioxyquinoléine par la 2-bromo-3-bromométhyl-4-cyclobutyl-6,7-méthylènedioxyquinoléine.

### PREPARATION 3 : 7-Ethyl-2,3-difluorométhylènedioxy-7-hydroxy-13-[3-pipéridinopropyl]-9,12-dihydro-7H-cyclopenta[6,7]-indolizino[1,2-b]quinoline-8,10-dione

Le composé du titre est préparé selon la méthode décrite dans l'Exemple 11 du brevet EP 1101765, en remplaçant la 2-bromo-3-bromométhyl-6,7-méthylènedioxyquinoléine par la 2-bromo-3-bromométhyl-4-pipéridinopropyl-6,7-diflurométhylènedioxyquinoléine.

### Microanalyse élémentaire :

| | C% | H% | N% |
|---|---|---|---|
| Calculé: | 64,80 | 5,44 | 7,82 |
| Trouvé: | 64,29 | 4,48 | 7,70 |

### PREPARATION 4 : 7-Ethyl-7-hydroxy-2,3-diflurométhylènedioxy-13-cyclobutyl-9,12-dihydro-7H-cyclopenta[6,7]indolizino[1,2-b]quinoline-8,10-dione

Le composé du titre est préparé selon la méthode décrite dans l'Exemple 11 du brevet EP 1101765, en remplaçant la 2-bromo-3-bromométhyl-6,7-méthylènedioxyquinoléine par la 2-bromo-3-bromométhyl-4-cyclobutyl-6,7-difluorométhylènedioxyquinoléine.

### Microanalyse élémentaire :

| | C% | H% | N% |
|---|---|---|---|
| Calculé: | 64,38 | 4,32 | 6,01 |
| Trouvé : | 63,15 | 4,46 | 5,76 |

### PREPARATION 5: 7-Ethyl-7-hydroxy-2,3-diflurométhylènedioxy-13-isopropyl-9,12-dihydro-7H-cyclopenta[6,7]indolizino[1,2-b]quinoline-8,10-dione

Le composé du titre est préparé selon la méthode décrite dans l'Exemple 11 du brevet EP 1101765, en remplaçant la 2-bromo-3-bromométhyl-6,7-méthylènedioxyquinoléine par la 2-bromo-3-bromométhyl-4-isopropyl-6,7-difluorométhylènedioxyquinoléine.

### Microanalyse élémentaire :

| | C% | H% | N% |
|---|---|---|---|
| Calculé: | 64,43 | 4,44 | 6,16 |
| Trouvé : | 63,50 | 4,70 | 6,29 |

### PREPARATION 6: 7-Ethyl-7-hydroxy-2,3-diflurométhylènedioxy-9,12-dihydro-7H-cyclopenta[6,7]indolizino[1,2-b]quinoline-8,10-dione

Le composé du titre est préparé selon la méthode décrite dans l'Exemple 11 du brevet EP 1101765, en remplaçant la 2-bromo-3-bromométhyl-6,7-méthylènedioxyquinoléine par la 2-bromo-3-bromométhyl-6,7-difluorométhylènedioxyquinoléine.

### Microanalyse élémentaire :

| | C% | H% | N% |
|---|---|---|---|
| Calculé: | 61,17 | 3,42 | 6,79 |
| Trouvé: | 59,78 | 3,30 | 6,58 |

### EXEMPLE 1 : Chlorhydrate de 3-piperidinopropanoate de 7-éthyl-2,3-méthylène-dioxy-13-méthyl-8,10-dioxo-8,9,10,12-tétrahydro-7H cyclopenta[6,7]-indolizino[1,2-b]quinolin-7-yl

A une suspension de 0,8 g (2 mmol) du composé de la préparation 1, dans 150 ml de dichlorométhane sont ajoutés successivement 1,13 g (7,2 mmol) d'acide 3-piperidin-1-ylpropanoïque, 2,28 g (12,7 mmol) de chlorhydrate de 1-(3-diméthylaminopropyl)-3-éthyl-carbodiimide, et 0,34 g (2,78 mmol) de 4-diméthylaminopyridine. Le milieu réactionnel est agité 24 heures à température ambiante, puis filtré. Le filtrat est lavé à l'aide d'une solution de bicarbonate de sodium puis à l'eau et séché sur sulfate de magnésium. Après concentration du solvant sous vide, le résidu est dissout dans une solution de dichlorométhane contenant 30% d'éthanol. 0,57 ml d'acide chlorhydrique 1N sont ajoutés et le précipité formé est filtré et recristallisé dans l'acétonitrile pour fournir le composé attendu.

### EXEMPLE 2 : Chlorhydrate de 3-piperidinopropanoate de 7-éthyl-2,3-méthylènedioxy-13'-cyclobuty]-8,10-dioxo-8,9,10,12-tétrabydro-7H-cyclopenta-[6,7]indolizino[1,2-b]quinolin-7-yl

Le composé du titre a été synthétisé comme décrit dans l'exemple 1 en remplaçant le produit de départ : composé de la préparation 1, par celui de la préparation 2.

### Spectre de masse : (MH⁺) m/z = 570,3

### EXEMPLE 3 : 3-Pipéridinopropanoate de 2,3-difluorométhylènedioxy-7-éthyl-8,10-dioxo-13-[3-(1-piperidinyl)propyl]-8,9,10,12-tetlrahydro-7H-cycopenta[6,7]indolizino[1,2-b]quinolin-7-yl

Le composé du titre a été synthétisé comme décrit dans l'exemple 1 en remplaçant le produit de départ : composé de la préparation 1, par celui de la préparation 3.

### EXEMPLE 4 : 3-Pipéridinopropanoate de 2,3-difluorométhylènedioxy-7-éthyl-8,10-dioxo-13-cyclobutyl-8,9,10,12-tétrahydro-7H-cyclopenta[6,7]-indolizino[1,2-b]quinolin-7-yl

Le composé du titre a été synthétisé comme décrit dans l'exemple 1 en remplaçant le produit de départ : composé de la préparation 1, par celui de la préparation 4.

### EXEMPLE 5 : 3-Pipéridinopropanoate de 2,3-difluorométhylènedioxy-7-éthyl-8,10-dioxo-13-isopropyl-8,9,10,12-tétrahydro-7H-cyclopenta[6,7]indolizino[1,2-b]quinolin-7-yl

Le composé du titre a été synthétisé comme décrit dans l'exemple 1 en remplaçant le produit de départ : composé de la préparation 1, par celui de la préparation 5.

### EXEMPLE 6 : 3-Pipéridinobutanoate de 2,3-difluorométhylènedioxy-7-éthyl-8,10-dioxo-8,9,10,12-tétrahydro-7H-cyclopenta[6,7]-indolizino[1,2-b]quinolin-7-yl

Le composé du titre a été synthétisé comme décrit dans l'exemple 1 en remplaçant l'acide 3-pipéridinopropanoïque par l'acide 4-pipéridinobutanoïque, et le produit de départ : composé de la préparation 1, par celui de la préparation 6.

Les composés des exemples 7 à 21 (voir infra) ont été obtenus en adaptant les protocoles expérimentaux 1 à 6 avec les substrats adéquats.

### EXEMPLE 7 : 3-Pipéridinopropanoate de 7-éthyl-2,3-difluoro-13-isopropyl-8,10-dioxo-8,9,10,12-tétrahydro-7H-cyclopenta[6,7]indolizino[1,2-b]-quinolin-7-yl

### EXEMPLE 8 : 3-Pipéridinopropanoate de 7-éthyl-2,3-difluoro-8-[2-(1,3dioxolan)yl]-13-isopropyl-10-oxo-8,9,10,12-tétrahydro-7H-cyclopenta[6,7]-indolizino[1,2-b]quinolin-7-yl

### EXEMPLE 9 : 3-Morpholinopropanoate de 13-13-[benzyl(méthyl)amino]propyl}-7-éthyl-2,3-difluoro-8,10-dioxo-8,9,10,12-tétrahydro-7H-cyclopenta[6,7]indolizino[1,2-b]quinolin-7-yl

### EXEMPLE 10 : 3-Diméthylaminopropanoate de 2,3-(difluorométhylènedioxy)-7-éthyl-8,10-dioxo-13-cyclobutyl-8,9,10,12-tétrahydro-7H-cyclopenta[6,7]indolizino[1,2-b]quinolin-7-yl

### EXEMPLE 11 : 3-Pipéridinobutanoate de 2,3-éthylènedioxy-7-éthyl-8,10-dioxo-13-méthoxyéthyl-8,9,1 0,1 2-tétrahydro-7H-cyclopenta[6,7]indolizino[1,2-b]quinolin-7-yl

### EXEMPLE 12 : 3-Pipéridinopropanoate de 2,3-éthylènedioxy-7-éthyl-8,10-dioxo-13-diméthylaminométhy-8,9,10,12-tétrahydro-7H-cyclopenta[6,7]-indolizino[1,2-b]quinolin-7-yl

### EXEMPLE 13 : 3-Pipéridinopropanoate de 2,3-méthylènedioxy-7-éthyl-8,10-dioxo-13-méthyl-8,9,10,12-tétrahydro-7H-cyclopenta[6,7]indolizino[1,2-b]quinolin-7-yl

### EXEMPLE 14 : 3-(4-Méthylpipérazino)propanoate de 3-chloro-7-éthyl-2-fluoro-8,9,10-trioxo-8,9,10,12-tétrahydro-7H-cyclopenta[6,7]indolizino[1,2-b]quinolin-7-yl

### EXEMPLE 15 : 3-(4-Méthylpipérazino)propanoate de 3-chloro-7-éthyl-2-fluoro-8,9,10-trioxo-8,9,1 0,12-tétrahydro-7H-cydopenta[6,7]indolizino[1,2-b]quinolin-7-yl

### EXEMPLE 16 : 3-Pipéridinopropanoate de 2,3-méthylènedioxy-7-éthyl-8,10-dioxo-13-cyclohexyl-8,9,10,12-tétrahydro-7H-cyclopenta-[6,7]indolizino[1,2-b]quinolin-7-yl

### EXEMPLE 17 : 3-(4-Méthylpiperazino)propanoate de 13-cyclobutyl-7-éthyl-2-fluoro-8,10-dioxo-3-(1-piperidinyl)-8,9,10,12-tétrahydro-7H-cyclopenta[6,7]-indolizino[1,2-b]quinolin-7-yl

### EXEMPLE 18 : 3-(4-Méthylpiperazino)propanoate de 13-(4-méthylpiperazinométhyl)-7-éthyl-2,3-éthylènedioxy-8,10-dioxo-8,9,10,12-tétrahydro-7H-cyclopenta[6,7]indolizino[1,2-b]quinolin-7-yl

### EXEMPLE 19 : 3-Pipéridinopropanoate de 3-chloro-7-éthyl-2-méthyl-8,9,10-trioxo-8,9,10,12-tétrahydro-7H-cyclopenta[6,7]indolizino[1,2-b]quinolin-7-yl

### EXEMPLE 20 : 3-Pipéridinopropanoate de 7-éthyl-2-hydroxy-8,10-dioxo-8,9,10,12-tétrahydro-7H-cyclopenta[6,7]indolizino[1,2-b]quinolin-7-yl

### EXEMPLE 21 : 3-Pipéridinopropanoate de 7-éthyl-2,3-méthylènedioxy-13-(2-méthyl-1-propènyl)-8,10-dioxo-8,9,10,12-tétrahydro-7H-cyclopenta[6,7]-indolizino[1,2-b]quinolin-7-yl

### EXEMPLE 22 : Chlorhydrate de 3-hexahydrocylopenta[c]pyrrol-2(1H)-ylpropanoate de 7-éthyl-2,3-méthylène-dioxy-13-cyclobutyl-8,10-dioxo-8,9,10,12-tétrahydro-7H-cylopenta-[6,7]indolizino[1,2-b]quinolin-7-yl

Le composé du titre a été synthétisé comme décrit dans l'exemple 1 en remplaçant l'acide 3-pipéridiiopropanoïque par l'acide 3-hexahydrocyclopenta[*c*]pyrrol-2(1*H*)-yl-propanoïque, et le produit de départ : composé de la préparation 1, par celui de la préparation 2.

### EXEMPLE 23 : Chlorhydrate de 3-[(4aR,8aS)-octahydroisoquinolin-2(1H)-yl]propanoate de 7-éthyl-2,3-méthylènedioxy-13-cyclobutyl-8,10-dioxo-8,9,10,12-tétrahydro-7H-cyclopenta-[6,7]indolizino[1,2-b]quinolin-7-yl

Le composé du titre a été synthétisé comme décrit dans l'exemple 1 en remplaçant l'acide 3-pipéridin-1-ylpropanoïque par l'acide 3-[(4aR,8aS)-octahydroisoquinolin-2(1*H*)-yl]-propanoïque, et le produit de départ : composé de la préparation 1, par celui de la préparation 2.

### EXEMPLE 24 : Chlorhydrate de 3-[(6,7-diméthoxy-3,4-dihydroisoquinolin-2(1H)-yl]propanoate de 7-éthyl-2,3-méthylènedioxy-13-cyclobutyl-8,10-dioxo-8,9,10,12-tëtrahydro-7H-cyclopenta-[6,7]indolizino[1,2-b]quinolin-7-yl

Le composé du titre a été synthétisé comme décrit dans l'exemple 1 en remplaçant l'acide 3-pipéridin-1-ylpropanoïque par l'acide 3-(6,7-diméthoxy-3,4-dihydroisoqionolin-2(1*H*)-yl)-propanoïque, et le produit de départ : composé de la préparation 1, par celui de la préparation 2.

### ETUDE PHARMACOLOGIQUE

### EXEMPLE A : Activité in vitro

La leucémie murine L1210 et les carcinomes humains du colon HCT116 et HT29 ont été utilisés *in vitro.* Les cellules sont cultivées dans du milieu de culture RPMI 1640 complet contenant 10 % de sérum de veau foetal, 2 mM de glutamine, 50 U/ml de péniciline, 50 µg/ml de streptomycine et 10 mM d'Hepes, pH : 7,4. Les cellules sont réparties dans des microplaques et exposées aux composés cytotoxiques pendant 4 temps de doublement, soit 48 heures (L1210) ou 96 heures (HCT116 et HT29). Le nombre de cellules viables est ensuite quantifié par un essai colorimétrique, le Microculture Tetrazolium Assay (J. Carmichael et al., Cancer Res. ; 47, 936-942, (1987)). Les résultats sont exprimés en IC₅₀, concentration en cytotoxique qui inhibe à 50 % la prolifération des cellules traitées.

Il apparaît que les composés de l'invention sont de puissants cytotoxiques, les IC₅₀ étant nettement inférieurs au µM.

| **Activité in vitro** | | | |
|---|---|---|---|
| | **IC₅₀ (nM)** | | |
| | **L1210** | **HCT116** | **HT29** |
| **Exemple 1** | 4.2 | - | - |
| **Exemple 2** | - | 1.5 | 3.7 |
| **Exemple 23** | 7.3 | 1.1 | 2.4 |
| **Exemple 24** | 7.9 | 0.7 | 2.6 |

### EXEMPLE B : Toxicité in vivo

Les produits sont formulés dans un mélange tween/eau, administrés par voie intraveineuse (i.v.) (administration pendant trois semaines, à raison d'une fois par semaine, le volume d'injection est de 0.2 ml /souris avec des doses croissantes de produits de 6,25 ; 12,5 ; 25 et 50 mg/kg) à des souris nude (bab/c fournies par Iffa Credo) pesant une 20^{aine} de gramme. La dose maximale tolérée (DMT) est la plus forte dose qui n'induit ni mortalité, ni perte de poids supérieure à 20%.

A titre d'exemple le composé de l'exemple 2 possède une DMT de 25 mg/lkg (administration intraveineuse, 1 fois par semaine pendant 3 semaines) soit deux fois moins toxique que son homologue proche structurellement « non estérifié » (composé de la Préparation 2) pour la même activité *in vivo* sur HCT116.

### EXEMPLE C : Composition pharmaceutique

Formule de préparation pour 1000 comprimés dosés à 10 mg :

| | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 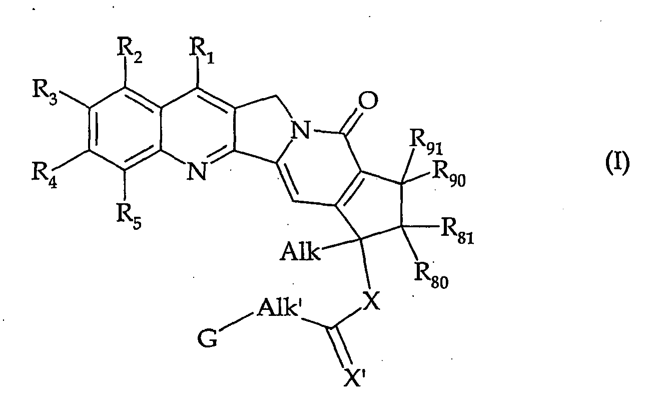 dans laquelle :
• Alk représente un groupement alkyle,
• R₁, R₂, R₃, R₄ et R₅ sont choisis indépendamment parmi un atome d'hydrogène, d'halogène, un groupement alkyle, alkényle, alkynyle, polyhalogénoalkyle, cycloalkyle éventuellement substitué, cycloalkylalkyle éventuellement substitué, aryle éventuellement substitué, hydroxy, hydroxyalkyle, alkoxy, alkoxyalkyle, nitro, cyano, acyloxy, -C(O)-R, et les groupements -(CH₂)ₚ-NRₐR_{b}, et -O-C(O)-N-RₐR_{b}, dans lesquels R représente un groupement alkyle, alkoxy ou amino(éventuellement substitué sur l'atome d'azote par un ou deux groupements alkyle), p est un entier compris entre 0 et 6, et Rₐ et R_{b} représentent indépendamment un atome d'hydrogène, un groupement alkyle, cycloalkyle, cycloalkylalkyle, acyle, aryle éventuellement substitué, arylalkyle éventuellement substitué, ou bien Rₐ et R_{b} forment ensemble avec l'atome d'azote qui les porte un groupement pyrrolyle, pipéridinyle, ou pipérazinyle, chacun de ces groupements cycliques pouvant être éventuellement substitué, ou bien deux groupements R₂, R₃, R₄ et R₅ adjacents forment ensemble avec les atomes de carbone qui les portent un groupement -T-(CR_{c}R_{d})ₜ-T'-, dans lequel T et T', identiques ou différents, représentent un atome d'oxygène, de souffre ou un groupement N-Rₑ ; R_{c} et R_{d}, identiques ou différents, représentent un atome d'hydrogène ou d'halogène ; t est un entier compris entre 1 et 3 inclus ; et Re représente un atome d'hydrogène, un groupement alkyle ou benzyle,
• R₈₀ et R₉₀ représentent indépendamment un atome d'hydrogène, un groupement hydroxy, alkyle, ou alkoxy,
• R₈₁ et R₉₁ représentent indépendamment un atome d'hydrogène, un groupement alkyle, alkényle, alkynyle, ou, pris deux à deux sur des carbones adjacents, forment ensemble une liaison, ou un groupement oxirane, ou bien deux groupements géminaux (R₈₀ et R₈₁) et/ou (R₉₀ et R₉₁) forment ensemble un groupement oxo ou un groupement -O-(CH₂)ₜ₁-O-, t₁ étant un entier compris entre 1 et 3 inclus,
• X et X', identiques ou différents, représentent un atome d'oxygène, de soufre, un groupement amino ou alkylamino,
• Alk' représente une chaîne alylène, alkénylène, ou alkynylène,
• G représente un groupement NR₆R₇ où:
i) soit R₆, R₇ représentent indépendamment l'un de l'autre un atome d'hydrogène, un groupement alkyle, cycloalkyle, aryle éventuellement substitué, arylalkyle éventuellement substitué, cycloalkyle éventuellement substitué, cycloalkylalkyle éventuellement substitué, hétéroaryle éventuellement substitué, ou hétéroarylalkyle éventuellement substitué,
ii) soit R₆ et R₇ forment ensemble avec l'atome d'azote un groupement hétérocycloalkyle monocyclique 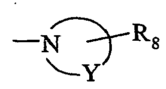 de 5 à 8 chaînons ou bicyclique 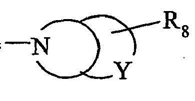 de 5 à 11 chaînons dons lesquels :
■ Y représente un atome d'azote, d'oxygène ou un groupement CH₂ et
■ R₈ représente un atome d'hydrogène, un groupement alkyle, cycloalkyle éventuellement substitué, cycloalkylalkyle éventuellement substitué, aryle éventuellement substitué, arylalkyle éventuellement substitué, hétérocycloalkyle éventuellement substitué, hétérocycloalkylalkyle éventuellement substitué, hétéroaryle éventuellement substitué, hétéroarylalkyle éventuellement substitué,
leurs énantiomères, diastéréoisomères, ainsi que leurs sels d'addition à un acide ou à une base pharmaceutiquement acceptable,
étant entendu que :
- le terme alkyle désigne une chaîne de 1 à 6 atomes de carbone, linéaire ou ramifié,
- lé terme alkényle désigne une chaîne de 2 à 6 atomes de carbone, linéaire ou ramifié et contenant de 1 à 3 doubles liaisons,
- le terme alkynyle désigne une chaîne de 2 à 6 atomes de carbone, linaire ou ramifié et contenant de 1 à 3 triples liaisons,
- le terme alkylène désigne un radical bivalent, linéaire ou ramifié, contenant de 1 à 6 atomes de carbone,
- le terme alkénylène désigne un radical bivalent, linéaire ou ramifié, contenant de 2 à 6 atomes de carbone et de 1 à 3 doubles liaisons,
- le terme alkynylène désigne un radical bivalent linéaire ou ramifié, contenant de 2 à 6 atomes de carbones et de 1 à 3 triples liaisons,
- le terme acyle désigne un radical alkyle-carbonyle linéaire ou ramifié contenant de 1 à 6 atomes de carbone,
- le terme alkoxy désigne un radical alkyle-oxy dont le groupement alkyle est linéaire ou ramifié, contenant de 1 à 6 atomes de carbone,
- le terme acyloxy désigne un radical acyle-oxy dont le groupement acyle est un radical akylcarbonyle linéaire ou ramifié,
- le terme aryloxyalkyle désigne un groupement aryle-oxy-alkyle dont le groupement alkyle est linéaire ou ramifié, contenant de 1 à 6 atomes de carbone,
- les termes arylalkyle, cycloalylalkyle, hétéroarylallcyle, hétérocycloalkylalkyle désignent les radicaux aryle-alkyle, cycloalkyle-alkyle, hétéroaryle-alkyle, hétérocycloalkyle-alkyle dans lesquels le groupement alkyle désigne une chaîne de 1 à 6 atomes de carbone, linéaire ou ramifié,
- le terme polyhalogénoalkyle désigne une chaîne carbonée, linéaire ou ramifiée, contenant de 1 à 3 atomes de carbone et de 1 à 7 atomes d'halogène,
- le terme halogène désigne les atomes de fluor, de chlore, de brome ou d'iode,
- le terme aryle désigne un groupement phényle, naphtyle, indanyle, indényle, dihydronaphtyle ou tétrahydronaphtyle,
- le terme cycloalkyle désigne un monocycle ou bicycle hydrocarboné comportant de 3 à 11 atomes de carbone, et éventuellement insaturé par 1 ou 2 insaturations,
- le terme hétéroaryle désigne un groupement monocyclique ou bicyclique dans lequel au moins un des cycles est aromatique, comportant de 5 à 11 chaînons et de 1 à 4 hétéroatomes, choisis parmi l'azote, l'oxygène et le soufre,
- le terme hétérocycloalkyle désigne un groupement mono ou bicyclique, saturé ou insaturé par 1 ou 2 insaturations, contenant de 4 à 11 chaînons et possédant de 1 à 4 hétéroatomes choisis parmi azote, oxygène et soufre,
- l'expression "éventuellement substitué" lorsqu'elle concerne les groupements aryle ou arylalkyle ; cycloalkyle ou cycloalkylalkyle ; hétéroaryle ou hétéroarylalkyle ; et hétérocycloalkyle ou hétérocycloalkylalkyle ; signifie que les groupements aryle; cycloalkyle ; hétéroaryle ; et hétérocycloalkyle respectivement peuvent être substitués par 1 à 3 substituants, identiques ou différents, choisis parmi l'atome d'halogène, groupement alkyle, alkoxy, alkylthio, alkylsulfinyle, alkylsulfonyle, hydroxy, mercapto, cyano, nitro, amino (éventuellement substitué par un ou deux groupements alkyle), acyle, formyle, aminocarbonyle (éventuellement substitué sur l'atome d'azote par un ou deux groupements alkyle), acylamino (éventuellement substitué sur l'atome d'azote par un groupement alkyle), alkoxycarbonyle, carboxy et sulfo,
- l'expression "éventuellement substitué" lorsqu'elle concerne les groupements pyrrolyle, pipéridinyle, ou pipérazinyle signifie que les groupements concernés peuvent être substitués par 1 à 3 groupements, identiques ou différents, choisis parmi alkyle, alkoxy, aryle, arylalkyle, aryloxy, et aryloxyalkyle.

2. Composés de formule (I) selon la revendication 1 pour lesquels Alk représente un groupement éthyle, leurs énantiomères, diastéréoisomères ainsi que leurs sels d'addition à un acide ou à une base pharmaceutiquement acceptable.

3. Composés de formule (I) selon la revendication 1 pour lesquels R₈₀ et R₈₁ forment ensemble un groupement oxo, ou bien R₉₀ et R₉₁ forment ensemble un groupement oxo, ou bien R₈₀ et R₈₁ ainsi que R₉₀ et R₉₁ forment deux groupements oxo, leurs énantiomères, diastéréoisomères ainsi que leurs sels d'addition à un acide ou à une base pharmaceutiquement acceptable.

4. Composés de formule (I) selon la revendication 1 pour lesquels R₅ représente un atome d'hydrogène, leurs énantiomères, diastéréoisomères ainsi que leurs sels d'addition à un acide ou à une base pharmaceutiquement acceptable.

5. Composés de formule (I) selon la revendication 1 pour lesquels R₂, R₃ et R₄ sont choisis parmi un atome d'hydrogène; d'halogène, un groupement alkyle ou alkoxy, leurs énantiomères, diastéréoisomères ainsi que leurs sels d'addition à un acide ou à une base pharmaceutiquement acceptable.

6. Composés de formule (I) selon la revendication 1 pour lesquels R₃ et R₄ forment ensemble un groupement méthylènedioxy ou éthylènedioxy, leurs énantiomères, diastéréoisomères ainsi que leurs sels d'addition à un acide ou à une base pharmaceutiquement acceptable.

7. Composés de formule (I) selon la revendication 1 pour lesquels R₂ représente un atome d'hydrogène, leurs énantiomères, diastéréoisomères ainsi que leurs sels d'addition à un acide ou à une base pharmaceutiquement acceptable.

8. Composés de formule (I) selon la revendication 1 pour lesquels R₁ représente un groupement alkyle, cycloalkyle ou cycloalkylalkyle, leurs énantiomères, diastéréoisomères ainsi que leurs sels d'addition à un acide ou à une base pharmaceutiquement acceptable.

9. Composés de formule (I) selon la revendication 1 pour lesquels R₁ représente un groupement aryle éventuellement substitué, leurs énantiomères, diastéréoisomères ainsi que leurs sels d'addition à un acide ou à une base pharmaceutiquement acceptable.

10. Composés de formule (1) selon la revendication 1 pour lesquels G représente un groupement NR₆R₇ où R₆ ef R₇ forment ensemble avec l'atome d'azote un groupement hétérocycloalkyle monocyclique 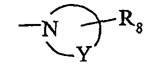 de 5 à 8 chaînons dans lequel Y représente un atome d'azote, d'oxygène ou un groupement CH₂ et R₈ représente un atome d'hydrogène ou un groupement alkyle, leurs énantiomères, diastéréoisomères ainsi que leurs sels d'addition à un acide ou à une base pharmaceutiquement acceptable.

11. Composés de formule (I) selon la revendication 1 pour lesquels Alk' représente un groupement alkylène, leurs énantiomères, diastéréoisomères ainsi que leurs sels d'addition à un acide ou à une base pharmaceutiquement acceptable.

12. Composés de formule (I) selon la revendication 1 pour lesquels X et X', identiques ou différents, représentent un atome d'oxygène ou de soufre, leurs énantiomères, diastéréoisomères ainsi que leurs sels d'addition à un acide ou à une base pharmaceutiquement acceptable.

13. Composé de formule (I) selon la revendication 1 qui est le 3-piperidinopropanoate de 7-éthyl-2,3-méthylènedioxy-13-méthyl-8,10-dioxo-8,9,10,12-tétra-hydro-7*H-*cyclopenta[6,7]indolizino[1,2-*b*]quinolin-7-yl, ses énantiomères ainsi que ses sels d'addition à un acide ou à une base pharmaceutiquement acceptable.

14. Composé de formule (I) selon la revendication 1 qui est le 3-piperidinopropanoate de 7-éthyl-2,3-méthylènedioxy-13-cyclobutyl-8,10-dioxo-8,9,10,12-tétrahydro-7*H-*cyclopenta[6,7]indolizino[1,2-*b*]quinolin-7-yl, ses énantiomères ainsi que ses sels d'addition à un acide ou à une base pharmaceutiquement acceptable.

15. Composé de formule (I) selon la revendication 1 qui est le 3-hexahydrocyclopenta[*c*]pyrrol-2(1*H*)-ylpropanoate de 7-éthyl-2,3-méthylène-dioxy-13-cyclobutyl-8,10-dioxo-8,9,10,12-tétrahydro-7*H*-cylopenta-[6,7]indolizino[1,2-*b*]quinolin-7-yl, ses énantiomères ainsi que ses sels d'addition à un acide ou à une base pharmaceutiquement acceptable.

16. Procédé de préparation des composés de formule (I) selon la revendication 1 **caractérisé en** en ce que l'on utilise comme produit de départ un composé de formule (II) synthétisé comme décrit dans EP 1101765 : 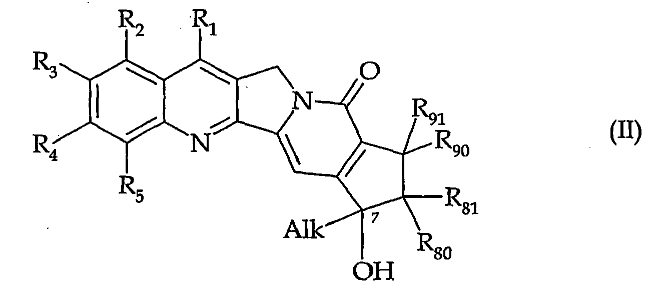 dans laquelle Alk, R₁, R₂, R₃, R₄, R₅ R₈₀, R₈₁, R₉₀ et R₉₁ sont tels que définis dans la formule (I),
dont le groupement hydroxy en C₇ est transformé en X"H avec X" représentant un groupement SH, amino, ou alkylamino pour conduire au composé de formule (III) 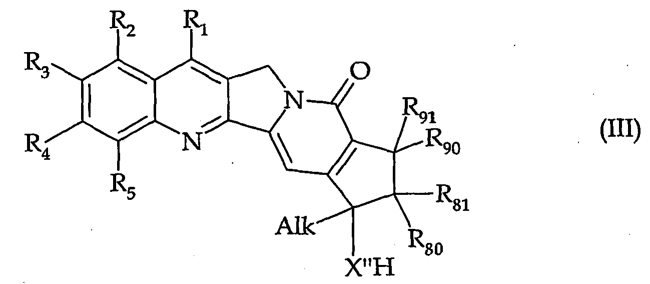 dans laquelle Alk, R₁, R₂, R₃, R₄, R₅ R₈₀, R₈₁, R₉₀ et R₉₁ sont tels que définis dans la formule (I), et X" est tel que défini précédemment,
composés de formule (II) ou (III) qui se condensent avec le réactif (IV) : 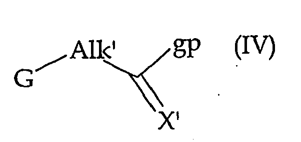 dans laquelle G, Alk' et X' sont tels que définis dans la formule (I), et gp un groupe partant tels que Hal, OH, SH, NR'R", OC(O)R' où R', R" représentent des groupements alkyle,
pour conduire au composé de formule (I),
étant entendu, dans le but de simplifier le procédé ci-dessus, que les groupements réactifs présents en R₈₀, R₈₁, R₉₀ et R₉₁ peuvent être protégés à l'aide de groupements protecteurs classiques et déprotégés au moment opportun, que les groupements hydroxy présents en ces mêmes positions peuvent être oxydés par des méthodes classiques de la chimie, en groupement oxo, qu'inversement les groupements oxo présents en ces mêmes positions peuvent être réduits par des réducteurs classiques, à tout moment opportun de la synthèse, et que lorsque deux de ces groupements forment ensemble une liaison, cette dernière peut être introduite à tout moment jugé utile par l'homme de métier afin de faciliter la synthèse,
composés de formule (I) :
- qui peuvent être, le cas échéant, purifiés selon une technique classique de purification,
- dont on sépare, le cas échéant, les stéréoisomères selon une technique classique de séparation,
- que l'on transforme, si on le souhaite, en leurs sels d'addition à un acide ou à une base pharmaceutiquement acceptable.

17. Composés de formule (III') : 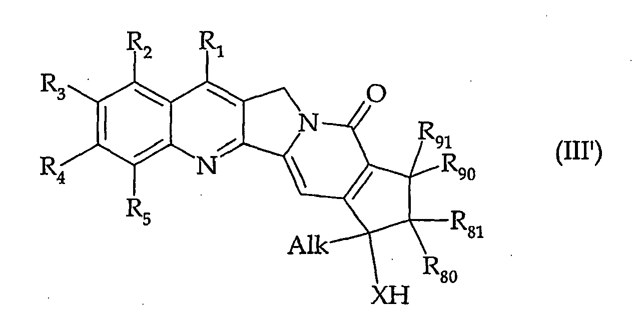 dans laquelle :
• Alk représente un groupement alkyle,
• R₁, R₂, R₃, R₄ et R₅ sont choisis indépendamment parmi un atome d'hydrogène, d'halogène, un groupement alkyle, alkényle, alkynyle, polyhalogénoalkyle, cycloalkyle éventuellement substitué, cycloalkylalkyle éventuellement substitué, hydroxy, hydroxyalkyle, alkoxy, alkoxyalkyle, nitro, cyano, acyloxy, -C(O)-R, et les groupements -(CH₂)ₚ-NRₐR_{b}, et -O-C(O)-N-RₐR_{b}, dans lesquels R représente un groupement alkyle, alkoxy ou amino(éventuellement substitué sur l'atome d'azote par un ou deux groupements alkyle), p est un entier compris entre 0 et 6, et Rₐ et R_{b} représentent indépendamment un atome d'hydrogène, un groupement alkyle, cycloalkyle, cycloalkylalkyle, acyle, aryle éventuellement substitué, arylalkyle éventuellement substitué, ou bien Rₐ et R_{b} forment ensemble avec l'atome d'azote qui les porte un groupement pyrrolyle, pipéridinyle, ou pipérazinyle, chacun de ces groupements cycliques pouvant être éventuellement substitué, et au moins deux groupements R₂, R₃, R₄ et R₅ adjacents forment ensemble avec les atomes de carbone qui les portent un groupement T-(CR_{c}R_{d})ₜ-T'-, dans lequel T et T', identiques ou différents, représentent un atome d'oxygène, de souffre ou un groupement N-Rₑ ; R_{c} et R_{d}, identiques ou différents, représentent un atome d'hydrogène ou d'halogène ; t est un entier compris entre 1 et 3 inclus ; et Rₑ représente un atome d'hydrogène, un groupement alkyle ou benzyle, étant entendu qu'au moins un des deux groupements R_{c} ou R_{d} représentent un atome d'halogène lorsque T et T' représentent chacun un atome d'oxygène et X représente un atome d'oxygène,
• R₈₀ et R₉₀ représentent indépendamment un atome d'hydrogène, un groupement hydroxy, alkyle, ou alkoxy,
• R₈₁ et R₉₁ représentent indépendamment un atome d'hydrogène, un groupement alkyle, alkényle, alkynyle, ou, pris deux à deux sur des carbones adjacents, forment ensemble une liaison, ou un groupement oxirane, ou bien deux groupements géminaux (R₈₀ et R₈₁) et/ou (R₉₀ et R₉₁) forment ensemble un groupement oxo ou un groupement -O-(CH₂)ₜ₁-O-, t₁ étant un entier compris entre 1 et 3 inclus,
• X représentent un atome d'oxygène, de souffre, un groupement amino ou alkylamino,
leurs énantiomères, diastéréoisomères, ainsi que leurs sels d'addition à un acide ou à une base pharmaceutiquement acceptable.

18. Compositions pharmaceutiques contenant comme principe actif au moins un composé selon l'une quelconque des revendications 1 à 15, seul ou en combinaison avec un ou plusieurs excipients ou véhicules inertes, non toxiques, pharmaceutiquement acceptables.

19. Compositions pharmaceutiques selon la revendication 18 contenant au moins un principe actif selon l'une quelconque des revendications 1 à 15 utiles pour la fabrication de médicaments utiles dans le traitement des maladies cancéreuses.

## Claims

1. Compounds of formula (I) : 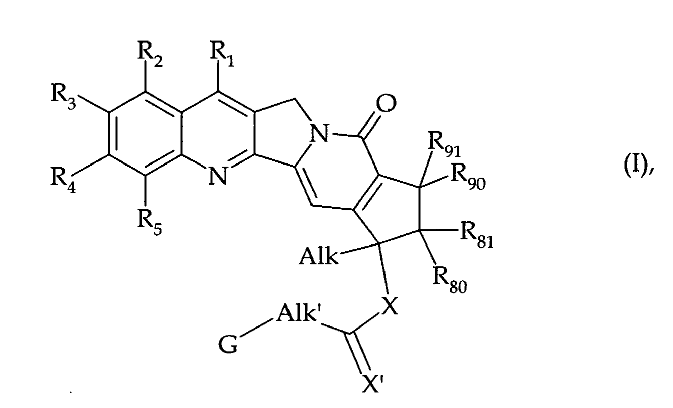 wherein :
• Alk represents an alkyl group,
• R₁, R₂, R₃, R₄ and R₅ are independently selected from a hydrogen atom, a halogen atom, an alkyl group, an alkenyl group, an alkynyl group, a polyhaloalkyl group, an optionally substituted cycloalkyl group, an optionally substituted cycloalkylalkyl group, an optionally substituted aryl group, a hydroxy group, a hydroxyalkyl group, an alkoxy group, an alkoxyalkyl group, a nitro group, a cyano group, an acyloxy group, a -C(O)-R group, and the groups -(CH₂)ₚ-NRₐR_{b} and -O-C(O)-N-RₐR_{b}, wherein R represents an alkyl group, an alkoxy group or an amino group (optionally substituted on the nitrogen atom by one or two alkyl groups), p is an integer from 0 to 6, and Rₐ and R_{b} independently represent a hydrogen atom, an alkyl group, a cycloalkyl group, a cycloalkylalkyl group, an acyl group, an optionally substituted aryl group or an optionally substituted arylalkyl group, or Rₐ and R_{b} form together with the nitrogen atom carrying them a pyrrolyl, piperidyl or piperazinyl group, it being possible for each of those cyclic groups to be optionally substituted,
or two adjacent groups from R₂, R₃, R₄ and R₅ form together with the carbon atoms carrying them a group -T-(CR_{c}R_{d})ₜ-T'-, wherein T and T', which are the same or different, represent an oxygen atom, a sulphur atom or a group N-Rₑ; R_{c} and R_{d}, which are the same or different, represent a hydrogen atom or a halogen atom; t is an integer from 1 to 3 inclusive; and Rₑ represents a hydrogen atom, an alkyl group or a benzyl group,
• R₈₀ and R₉₀ independently represent a hydrogen atom, a hydroxy group, an alkyl group or an alkoxy group,
• R₈₁ and R₉₁ independently represent a hydrogen atom, an alkyl group, an alkenyl group or an alkynyl group, or, taken in pairs on adjacent carbon atoms, together form a bond or an oxirane group, or two geminal groups (R₈₀ and R₈₁) and/or (R₉₀ and R₉₁) together form an oxo group or a group -O-(CH₂)ₜ₁-O-, t₁ being an integer from 1 to 3 inclusive,
• X and X', which are the same or different, represent an oxygen atom, a sulphur atom, an amino group or an alkylamino group,
• Alk' represents an alkylene, alkenylene or alkynylene chain,
• G represents a group NR₆R₇ wherein:
i) either R₆ and R₇ represent, each independently of the other, a hydrogen atom, an alkyl group, a cycloalkyl group, an optionally substituted aryl group, an optionally substituted arylalkyl group, an optionally substituted cycloalkyl group, an optionally substituted cycloalkylalkyl group, an optionally substituted heteroaryl group or an optionally substituted heteroarylalkyl group,
ii) or R₆ and R₇ form together with the nitrogen atom a 5- to 8-membered monocyclic heterocycloalkyl group 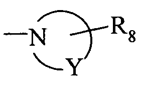 or a 5- to 11-membered bicyclic heterocycloalkyl group 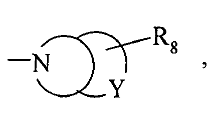 wherein:
■ Y represents a nitrogen atom, an oxygen atom or a CH₂ group and
■ R₈ represents a hydrogen atom, an alkyl group, an optionally substituted cycloalkyl group, an optionally substituted cycloalkylalkyl group, an optionally substituted aryl group, an optionally substituted arylalkyl group, an optionally substituted heterocycloalkyl group, an optionally substituted heterocycloalkylalkyl group, an optionally substituted heteroaryl group or an optionally substituted heteroarylalkyl group,
their enantiomers and diastereoisomers, and addition salts thereof with a pharmaceutically acceptable acid or base,
it being understood that:
- the term alkyl denotes a linear or branched chain of from 1 to 6 carbon atoms,
- the term alkenyl denotes a linear or branched chain of from 2 to 6 carbon atoms containing from 1 to 3 double bonds,
- the term alkynyl denotes a linear or branched chain of from 2 to 6 carbon atoms containing from 1 to 3 triple bonds,
- the term alkylene denotes a linear or branched divalent radical containing from 1 to 6 carbon atoms,
- the term alkenylene denotes a linear or branched divalent radical containing from 2 to 6 carbon atoms and from 1 to 3 double bonds,
- the term alkynylene denotes a linear or branched divalent radical containing from 2 to 6 carbon atoms and from 1 to 3 triple bonds,
- the term acyl denotes a linear or branched alkyl-carbonyl radical containing from 1 to 6 carbon atoms,
- the term alkoxy denotes an alkyl-oxy radical, the alkyl group of which is linear or branched and contains from 1 to 6 carbon atoms,
- the term acyloxy denotes an acyl-oxy radical, the acyl group of which is a linear or branched alkylcarbonyl radical,
- the term aryloxyalkyl denotes an aryl-oxy-alkyl group, the alkyl group of which is linear or branched and contains from 1 to 6 carbon atoms,
- the terms arylalkyl, cycloalkylalkyl, heteroarylalkyl and heterocycloalkylalkyl denote aryl-alkyl, cycloalkyl-alkyl, heteroaryl-alkyl and heterocycloalkyl-alkyl radicals, the alkyl groups of which denote a linear or branched chain of from 1 to 6 carbon atoms,
- the term polyhaloalkyl denotes a linear or branched carbon chain containing from 1 to 3 carbon atoms and from 1 to 7 halogen atoms,
- the term halogen denotes fluorine, chlorine, bromine or iodine atoms,
- the term aryl denotes a phenyl, naphthyl, indanyl, indenyl, dihydronaphthyl or tetrahydronaphthyl group,
- the term cycloalkyl denotes a monocyclic or bicyclic hydrocarbon group containing from 3 to 11 carbon atoms and optionally being unsaturated with 1 or 2 unsaturated bonds,
- the term heteroaryl denotes a monocyclic or bicyclic group wherein at least one of the rings is aromatic, containing from 5 to 11 ring members and containing from 1 to 4 hetero atoms selected from nitrogen, oxygen and sulphur,
- the term heterocycloalkyl denotes a mono- or bi-cyclic group which is saturated or unsaturated with 1 or 2 unsaturated bonds, containing from 4 to 11 ring members and containing from 1 to 4 hetero atoms selected from nitrogen, oxygen and sulphur,
- the expression "optionally substituted" when used in relation to aryl or arylalkyl, cycloalkyl or cycloalkylalkyl, heteroaryl or heteroarylalkyl, and heterocycloalkyl or heterocycloalkylalkyl groups means that the respective aryl, cycloalkyl, heteroaryl and heterocycloalkyl groups may be substituted by from 1 to 3 identical or different substituents selected from a halogen atom and the groups alkyl, alkoxy, alkylthio, alkylsulphinyl, alkylsulphonyl, hydroxy, mercapto, cyano, nitro, amino (optionally substituted by one or two alkyl groups), acyl, formyl, aminocarbonyl (optionally substituted on the nitrogen atom by one or two alkyl groups), acylamino (optionally substituted on the nitrogen atom by an alkyl group), alkoxycarbonyl, carboxy and sulpho,
- the expression "optionally substituted" when used in relation to the groups pyrrolyl, piperidyl or piperazinyl means that the groups concerned may be substituted by from 1 to 3 identical or different groups selected from alkyl, alkoxy, aryl, arylalkyl, aryloxy and aryloxyalkyl.

2. Compounds of formula (I) according to claim 1, wherein Alk represents an ethyl group, their enantiomers and diastereoisomers, and addition salts thereof with a pharmaceutically acceptable acid or base.

3. Compounds of formula (I) according to claim 1, wherein R₈₀ and R₈₁ together form an oxo group, or wherein R₉₀ and R₉₁ together form an oxo group, or wherein R₈₀ and R₈₁ and also R₉₀ and R₉₁ form two oxo groups, their enantiomers and diastereoisomers, and addition salts thereof with a pharmaceutically acceptable acid or base.

4. Compounds of formula (I) according to claim 1, wherein R₅ represents a hydrogen atom, their enantiomers and diastereoisomers, and addition salts thereof with a pharmaceutically acceptable acid or base.

5. Compounds of formula (I) according to claim 1, wherein R₂, R₃ and R₄ are selected from a hydrogen atom, a halogen atom, an alkyl group and an alkoxy group, their enantiomers and diastereoisomers, and addition salts thereof with a pharmaceutically acceptable acid or base.

6. Compounds of formula (I) according to claim 1, wherein R₃ and R₄ together form a methylenedioxy or ethylenedioxy group, their enantiomers and diastereoisomers, and addition salts thereof with a pharmaceutically acceptable acid or base.

7. Compounds of formula (I) according to claim 1, wherein R₂ represents a hydrogen atom, their enantiomers and diastereoisomers, and addition salts thereof with a pharmaceutically acceptable acid or base.

8. Compounds of formula (I) according to claim 1, wherein R₁ represents an alkyl, cycloalkyl or cycloalkylalkyl group, their enantiomers and diastereoisomers, and addition salts thereof with a pharmaceutically acceptable acid or base.

9. Compounds of formula (I) according to claim 1, wherein R₁ represents an optionally substituted aryl group, their enantiomers and diastereoisomers, and addition salts thereof with a pharmaceutically acceptable acid or base.

10. Compounds of formula (I) according to claim 1, wherein G represents a group NR₆R₇ wherein R₆ and R₇ form together with the nitrogen atom a 5- to-8-membered monocyclic heterocycloalkyl group 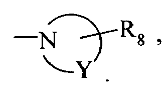 wherein Y represents a nitrogen atom, an oxygen atom or a group CH₂ and R₈ represents a hydrogen atom or an alkyl group, their enantiomers and diastereoisomers, and addition salts thereof with a pharmaceutically acceptable acid or base.

11. Compounds of formula (I) according to claim 1, wherein Alk' represents an alkylene group, their enantiomers and diastereoisomers, and addition salts thereof with a pharmaceutically acceptable acid or base.

12. Compounds of formula (I) according to claim 1, wherein X and X', which are the same or different, represent an oxygen atom or a sulphur atom, their enantiomers and diastereoisomers, and addition salts thereof with a pharmaceutically acceptable acid or base.

13. Compound of formula (I) according to claim 1, which is 7-ethyl-2,3-methylenedioxy-13-methyl-8,10-dioxo-8,9,10,12-tetrahydro-7*H*-cyclopenta[6,7]-indolizino[1,2-b]quinolin-7-yl 3-piperidinopropanoate, its enantiomers and addition salts thereof with a pharmaceutically acceptable acid or base.

14. Compound of formula (I) according to claim 1, which is 7-ethyl-2,3-methylenedioxy-13-cyclobutyl-8,10-dioxo-8,9,10,12-tetrahydro-7*H*-cyclopenta[6,7]-indolizino[1,2-*b*]quinolin-7-yl 3-piperidinopropanoate, its enantiomers and addition salts thereof with a pharmaceutically acceptable acid or base.

15. Compound of formula (I) according to claim 1, which is 7-ethyl-2,3-methylenedioxy-13-cyclobutyl-8,10-dioxo-8,9,10,12-tetrahydro-7*H*-cyclopenta[6,7]indolizino-[1,2-*b*]quinolin-7-yl 3-hexahydrocyclopenta[*c*]pyrrol-2(1*H*)-ylpropanoate, its enantiomers and addition salts thereof with a pharmaceutically acceptable acid or base.

16. Process for the preparation of compounds of formula (I) according to claim 1, **characterised in that** there is used as starting material a compound of formula (II) synthesised as described in EP 1 101 765 : 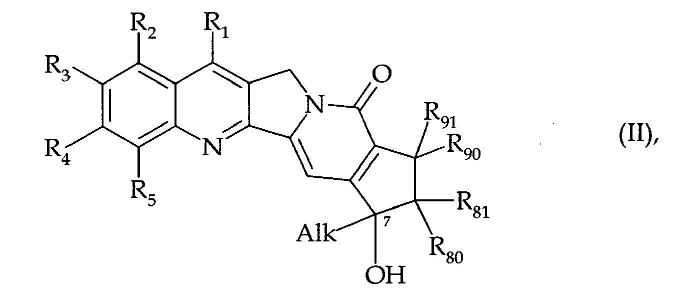 wherein Alk, R₁, R₂, R₃, R₄, R₅, R₈₀, R₈₁, R₉₀ and R₉₁ are as defined for formula (I),
wherein the hydroxy group at C₇ is converted into X"H wherein X" represents an SH, amino or alkylamino group to yield the compound of formula (III) 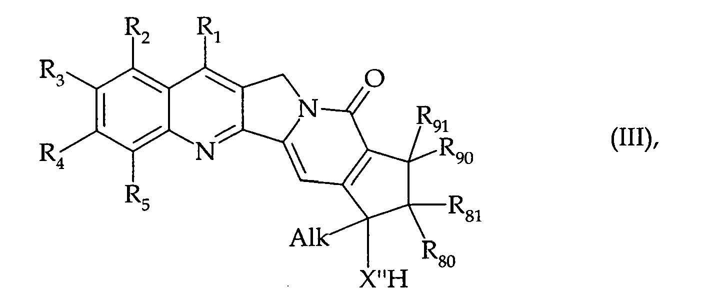 wherein Alk, R₁, R₂, R₃, R₄, R₅, R₈₀, R₈₁, R₉₀ and R₉₁ are as defined for formula (I) and X" is as defined hereinbefore,
which compounds of formula (II) or (III) are condensed with the reagent (IV) : 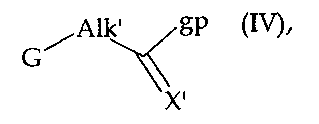 wherein G, Alk' and X' are as defined for formula (I) and gp is a leaving group such as Hal, OH, SH, NR'R" or OC(O)R' wherein R' and R" represent alkyl groups,
to yield the compound of formula (I),
it being understood, for the purpose of simplifying the above process, that the reactive groups present in R₈₀, R₈₁, R₉₀ and R₉₁ may be protected by conventional protecting groups and deprotected at the appropriate point in time, that the hydroxy groups present in those same positions may be oxidised to oxo groups by conventional chemistry methods, and, conversely, the oxo groups present in those same positions may be reduced by conventional reducing agents at any appropriate point in time during synthesis, and that, when two of those groups together form a bond, the latter can be introduced at any point in time deemed useful by the person skilled in the art in order to facilitate synthesis,
which compounds of formula (I) :
- may be purified, if necessary, according to a conventional purification technique,
- are separated, where appropriate, into their stereoisomers according to a conventional separation technique,
- are converted, if desired, into addition salts thereof with a pharmaceutically acceptable acid or base.

17. Compounds of formula (III'): 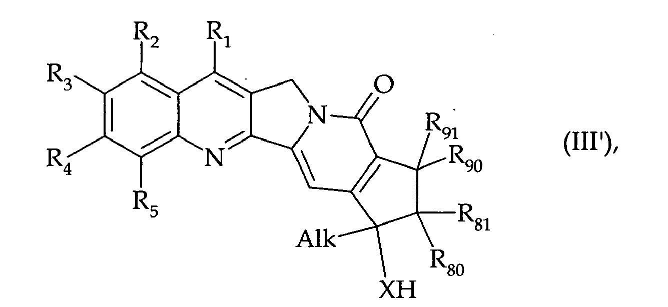 wherein:
• Alk represents an alkyl group,
• R₁, R₂, R₃, R₄ and R₅ are independently selected from a hydrogen atom, a halogen atom, an alkyl group, an alkenyl group, an alkynyl group, a polyhaloalkyl group, an optionally substituted cycloalkyl group, an optionally substituted cycloalkylalkyl group, a hydroxy group, a hydroxyalkyl group, an alkoxy group, an alkoxyalkyl group, a nitro group, a cyano group, an acyloxy group, a -C(O)-R group, and the groups -(CH₂)ₚ-NRₐR_{b} and -O-C(O)-N-RₐR_{b}, wherein R represents an alkyl group, an alkoxy group or an amino group (optionally substituted on the nitrogen atom by one or two alkyl groups), p is an integer from 0 to 6, and Rₐ and R_{b} independently represent a hydrogen atom, an alkyl group, a cycloalkyl group, a cycloalkylalkyl group, an acyl group, an optionally substituted aryl group or an optionally substituted arylalkyl group, or Rₐ and R_{b} form together with the nitrogen atom carrying them a pyrrolyl, piperidyl or piperazinyl group, it being possible for each of those cyclic groups to be optionally substituted, and at least two adjacent groups from R₂, R₃, R₄ and R₅ form together with the carbon atoms carrying them a group -T-(CR_{c}R_{d})ₜ-T'-, wherein T and T', which are the same or different, represent an oxygen atom, a sulphur atom or a group N-Rₑ; R_{c} and R_{d}, which are the same or different, represent a hydrogen atom or a halogen atom; t is an integer from 1 to 3 inclusive; and Rₑ represents a hydrogen atom, an alkyl group or a benzyl group, it being understood that at least one of the two groups R_{c} or R_{d} represents a halogen atom when T and T' each represent an oxygen atom and X represents an oxygen atom,
• R₈₀ and R₉₀ independently represent a hydrogen atom, a hydroxy group, an alkyl group or an alkoxy group,
• R₈₁ and R₉₁ independently represent a hydrogen atom, an alkyl group, an alkenyl group or an alkynyl group, or, taken in pairs on adjacent carbon atoms, together form a bond or an oxirane group, or two geminal groups (R₈₀ and R₈₁) and/or (R₉₀ and R₉₁) together form an oxo group or a group -O-(CH₂)ₜ₁-O-, t₁ being an integer from 1 to 3 inclusive,
• X represents an oxygen atom, a sulphur atom, an amino group or an alkylamino group,
their enantiomers and diastereoisomers, and addition salts thereof with a pharmaceutically acceptable acid or base.

18. Pharmaceutical compositions comprising as active ingredient at least one compound according to any one of claims 1 to 15, alone or in combination with one or more inert, non-toxic, pharmaceutically acceptable excipients or carriers.

19. Pharmaceutical compositions according to claim 18 comprising at least one active ingredient according to any one of claims 1 to 15 for use in the manufacture of medicaments for use in the treatment of cancer diseases.

## Patentansprüche

1. Verbindungen der Formel (1): 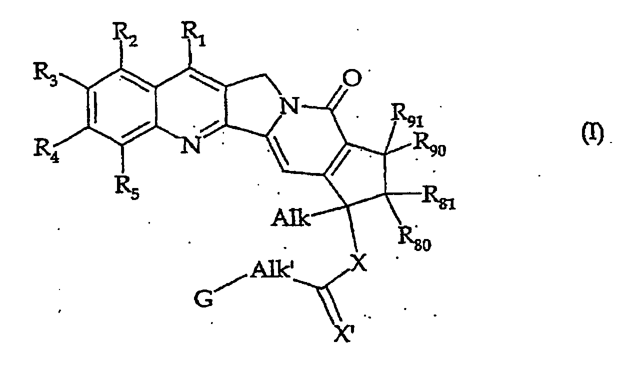 in der:
• Alk eine Alkylgruppe bedeutet,
• R₁, R₂, R₃, R₄ und R₅ unabhängig voneinander ausgewählt sind aus Wasserstoffatomen oder Halogenatomen, Alkyl-, Alkenyl-, Alkinyl-, Polyhalogenalkyl-, gegebenenfalls substituierten Cycloalkyl-, gegebenenfalls substituierten Cycloalkylalkyl-, gegebenenfalls substituierten Aryl-, Hydroxy-, Hydroxyalkyl-, Alkoxy-, Alkocyalkyl-, Nitro-, Cyano-, Acyloxy-gruppen, Gruppen -C(O)-R und Gruppen -(CH₂)ₚ-NRₐR_{b} und -O-C(O)-N-RₐR_{b}, worin R eine Alkyl-, Alkoxy- oder (gegebenenfalls am Stickstoffatom durch eine oder zwei Alkylgruppen substituierte) Aminogruppe bedeutet, p eine ganze Zahl mit einem Wert zwischen 0 und 6 darstellt und Rₐ und R_{b} unabhängig voneinander ein Wasserstoffatom, eine Alkyl-, Cycloalkyl-, Cycloalkylalkyl-, Acyl-, gegebenenfalls substituierte Aryl- oder gegebenenfalls substituierte Arylalkylgruppe bedeuten oder Rₐ und R_{b} gemeinsam mit dem sie tragenden Stickstoffatom eine Pyrrolyl-, Piperidinyl- oder Piperazinylgruppe bedeuten, wobei jede dieser cyclischen Gruppen gegebenenfalls substituiert sein kann,
oder zwei benachbarte Gruppen R₂, R₃, R₄ und R₅ gemeinsam mit den sie tragenden Kohlenstoffatomen eine Gruppe -T-(CR_{c}R_{d})ₜ-T'- bilden, worin T und T', die gleichartig oder verschieden sind, ein Sauerstoffatom, ein Schwefelatom oder eine Gruppe N-Rₑ darstellen; R_{c} und R_{d}, die gleichartig oder verschieden sind, ein Wasserstoffatom oder ein Halogenatom bedeuten; t eine ganze Zahl mit einem Wert zwischen 1 und 3 einschließlich bedeutet; und Re ein Wasserstoffatom, eine Alkylgruppe oder eine Benzylgruppe bedeutet,
• R₈₀ und R₉₀ unabhängig voneinander ein Wasserstoffatom, eine Hydroxy-, Alkyl- oder Alkoxygruppe bedeuten,
• R₈₁ und R₉₁ unabhängig voneinander ein Wasserstoffatom, eine Alkyl-, Alkenyl- oder Alkinylgruppe bedeuten oder jeweils zwei Gruppen an benachbarten Kohlenstoffatomen gemeinsam eine Bindung bilden oder eine Oxirangruppe oder zwei geminale Gruppen (R₈₀ und R₈₁) und/oder (R₉₀ und R₉₁) gemeinsam eine Oxogruppe oder eine Gruppe -O-(CH₂)ₜ₁-O- bilden, wobei t₁ eine ganze Zahl mit einem Wert zwischen 1 und 3 einschließlich bedeutet,
• X und X', die gleichartig oder verschieden sind, ein Sauerstoff- oder Schwefelatom oder eine Amino- oder Alkylaminogruppe bedeuten,
• Alk' eine Alkylen-, Alkenylen- oder Alkinylengruppe darstellt,
• G eine Gruppe NR₆R₇ bedeutet, worin:
i) entweder R₆ und R₇ unabhängig voneinander ein Wasserstoffatom, eine Alkyl-, Cycloalkyl-, gegebenenfalls substituierte Aryl-, gegebenenfalls substituierte Arylalkyl-, gegebenenfalls substituierte Cycloalkyl-, gegebenenfalls substituierte Cycloalkylalkyl-, gegebenenfalls substituierte Heteroaryl- oder gegebenenfalls substituierte Heteroarylalkylgruppe bedeuten,
ii) oder R₆ und R₇ gemeinsam mit dem Stickstoffatom eine monocyclische Heterocycloalkylgruppe der Formel 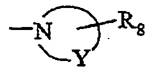 mit 5 bis 8 Kettengliedern oder bicyclische Heterocycloalkylgruppe der Formel 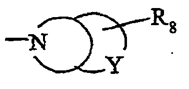 mit 5 bis 11 Ketgliedern bilden, worin:
■ Y ein Stickstoffatom, ein Sauerstoffatom oder eine Gruppe CH₂ bedeutet und
■ R₈ ein Wasserstoffatom, eine Alkyl-, gegebenenfalls substituierte Cycloalkyl-, gegebenenfalls substituierte Cycloalkylalkyl-, gegebenenfalls substituierte Aryl-, gegebenenfalls substituierte Arylalkyl-, gegebenenfalls substituierte Heterocycloalkyl-, gegebenenfalls substituierte Heterocycloalkylalkyl-, gegebenenfalls substituierte Heteroaryl- oder gegebenenfalls substituierte Heteroarylalkylgruppe bedeutet,
deren Enantiomere, Diastereoisomere sowie deren Additionssalze mit einer pharmazeutisch annehmbaren Säure oder Base,
wobei es sich versteht, dass:
- der Begriff Alkyl eine geradkettige oder verzweigte Kette mit 1 bis 6 Kohlenstoffatomen bezeichnet,
- der Begriff Alkenyl eine geradkettige oder verzweigte Kette mit 2 bis 6 Kohlenstoffatomen bezeichnet, die 1 bis 3 Doppelbindungen enthält,
- der Begriff Alkinyl eine geradkettige oder verzweigte Kette mit 2 bis 6 Kohlenstoffatomen bezeichnet, die 1 bis 3 Dreifachbindungen enthält,
- der Begriff Alkylen einen geradkettigen oder verzweigten zweiwertigen Rest bezeichnet, der 1 bis 6 Kohlenstoffatome enthält,
- der Begriff Alkenylen einen geradkettigen oder verzweigten zweiwertigen Rest bezeichnet, der 2 bis 6 Kohlenstoffatome und 1 bis 3 Doppelbindungen enthält,
- der Begriff Alkinylen einen geradkettigen oder verzweigten zweiwertigen Rest bezeichnet, der 2 bis 6 Kohlenstoffatome und 1 bis 3 Dreifachbindungen enthält,
- der Begriff Acyl einen geradkettigen oder verzweigten Alkyl-carbonyl-Rest bezeichnet, der 1 bis 6 Kohlenstoffatome enthält,
- der Begriff Alkoxy einen Alkyl-oxy-Rest bezeichnet, dessen Alkylgruppe geradkettig oder verzweigt ist und 1 bis 6 Kohlenstoffatome enthält,
- der Begriff Acyloxy einen Acyl-oxy-Rest bezeichnet, dessen Acylgruppe ein geradkettiger oder verzweigter Alkylcarbonyl-Rest ist,
- der Begriff Aryloxyalkyl eine Aryl-oxy-alkyl-Gruppe bezeichnet, deren Alkylgruppe geradkettig oder verzweigt ist und 1 bis 6 Kohlenstoffatome enthält,
- die Begriffe Aryalkyl, Cycloalkylalkyl, Heteroarylalkyl und Heterocycloalkylalkyl Aryl-alkyl-, Cycloalkyl-alkyl-, Heteroaryl-alkyl-, Heterocycloalkyl-alkyl-Reste bezeichnen, bei denen die Alkylgruppe eine geradkettige oder verzweigte Kette mit 1 bis 6 Kohlenstoffatomen darstellt,
- der Begriff Polyhalogenalkyl eine geradkettige oder verzweigte kohlenstoffhaltige Kette bezeichnet, die 1 bis 3 Kohlenstoffatome und 1 bis 7 Halogenatome enthält,
- der Begriff Halogen die Fluor-, Chlor- Brom- oder Iodatome bezeichnet,
- der Begriff Aryl eine Phenyl-, Naphthyl-, Indanyl-, Indenyl-, Dihydronaphthyl- oder Tetrahydronaphthylgruppe bezeichnet,
- der Begriff Cycloalkyl einen monocyclischen oder bicyclischen Kohlenwasserstoff bezeichnet, der 3 bis 11 Kohlenstoffatome aufweist und gegebenenfalls durch eine oder zwei Unsättigungen ungesättigt ist,
- der Begriff Heteroaryl eine monocyclische oder bicyclische Gruppe bezeichnet, bei der mindestens einer der Ringe aromatisch ist und die 5 bis 11 Kettenglieder und 1 bis 4 Heteroatome ausgewählt aus Stickstoff, Sauerstoff und Schwefel enthält,
- der Begriff Heterocycloalkyl eine mono- oder bicyclische Gruppe bezeichnet, die gesättigt oder durch 1 oder 2 Unsättigungen ungesättigt ist und 4 bis 11 Kettenglieder aufweist und 1 bis 4 Heteroatome ausgewählt aus Stickstoff, Sauerstoff und Schwefel umfasst,
- der Begriff "gegebenenfalls substituiert", wenn er die Aryl- oder Arylalkyl-; Cycloalkyl- oder Cycloalkylalkyl-; Heteroaryl- oder Heteroarylalkyl-; und Heterocycloalkyl- oder Heterocycloalkylalkylgruppen betrifft, bedeutet, dass die Aryl-; Cycloalkyl-; Heteroaryl-; bzw. Heterocycloalkylgruppen durch 1 bis 3 gleichartige oder verschiedenartige Substituenten substituiert sein können, ausgewählt aus Halogenatomen, Alkyl-, Alkoxy-, Alkylthio-, Alkylsulfinyl-, Alkylsulfonyl-, Hydroxy-, Mercapto-, Cyano-, Nitro-, (gegebenenfalls durch eine oder zwei Alkylgruppen substituierte) Amino-, Acyl-, Formyl-, (gegebenenfalls am Stickstoffatom durch eine oder zwei Alkylgruppen substituierte) Aminocarbonyl-, (gegebenenfalls am Stickstoffatom durch eine Alkylgruppe substituierte) Acylamino-, Alkoxycarbonyl-, Carboxy- und Sulfogruppen substituiert sein können,
- der Begriff "gegebenenfalls substituiert", wenn er die Pyrrolyl-, Piperidinyl- oder Piperazinylgruppen betrifft, bedeutet, dass die betreffenden Gruppen durch 1 bis 3 gleichartige oder verschiedenartige Gruppen ausgewählt aus Alkyl, Alkoxy, Aryl, Arylalkyl, Aryloxy und Aryloxyalkyl substituiert sein können.

2. Verbindungen der Formel (I) nach Anspruch 1, worin Alk eine Ethylgruppe bedeutet, deren Enantiomere, Diastereoisomere sowie deren Additionssalze mit einer pharmazeutisch annehmbaren Säure oder Base.

3. Verbindungen der Formel (I) nach Anspruch 1, worin R₈₀ und R₈₁ gemeinsam eine Oxogruppe bilden oder R₉₀ und R₉₁ gemeinsam eine Oxogruppe bilden oder R₈₀ und R₈₁ sowie R₉₀ und R₉₁ zwei Oxogruppen bilden, deren Enantiomere, Diastereoisomere sowie deren Additionssalze mit einer pharmazeutisch annehmbaren Säure oder Base.

4. Verbindungen der Formel (I) nach Anspruch 1, worin R₅ ein Wasserstoffatom bedeutet, deren Enantiomere, Diastereoisomere sowie deren Additionssalze mit einer pharmazeutisch annehmbaren Säure oder Base.

5. Verbindungen der Formel (I) nach Anspruch 1, worin R₂, R₃ und R₄ aus Wasserstoffatomen, Halogenatomen, Alkyl- oder Alkoxygruppen ausgewählt sind, deren Enantiomere, Diastereoisomere sowie deren Additionssalze mit einer pharmazeutisch annehmbaren Säure oder Base.

6. Verbindungen der Formel (I) nach Anspruch 1, worin R₃ und R₄ gemeinsam eine Methylendioxy- oder Ethylendioxygruppe bilden, deren Enantiomere, Diastereoisomere sowie deren Additionssalze mit einer pharmazeutisch annehmbaren Säure oder Base.

7. Verbindungen der Formel (I) nach Anspruch 1, worin R₂ ein Wasserstoffatom bedeutet, deren Enantiomere, Diastereoisomere sowie deren Additionssalze mit einer pharmazeutisch annehmbaren Säure oder Base.

8. Verbindungen der Formel (I) nach Anspruch 1, worin R₁ eine Alkyl-, Cycloalkyl- oder Cycloalkylalkylgruppe bedeutet, deren Enantiomere, Diastereoisomere sowie deren Additionssalze mit einer pharmazeutisch annehmbaren Säure oder Base.

9. Verbindungen der Formel (I) nach Anspruch 1, worin R₁ eine gegebenenfalls substituierte Arylgruppe bedeutet, deren Enantiomere, Diastereoisomere sowie deren Additionssalze mit einer pharmazeutisch annehmbaren Säure oder Base.

10. Verbindungen der Formel (I) nach Anspruch 1, worin G eine Gruppe NR₆R₇ bedeutet, worin R₆ und R₇ gemeinsam mit dem Stickstoffatom eine monocyclische Heterocycloalkylgruppe der Formel 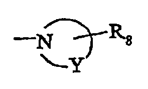 mit 5 bis 8 Kettengliedern bilden, worin Y ein Stickstoffatom, Sauerstoffatom oder eine Gruppe CH₂ und R₈ ein Wasserstoffatom oder eine Alkylgruppe bedeuten, deren Enantiomere, Diastereoisomere sowie deren Additionssalze mit einer pharmazeutisch annehmbaren Säure oder Base.

11. Verbindungen der Formel (I) nach Anspruch 1, worin Alk' eine Alkylengruppe bedeutet, deren Enantiomere, Diastereoisomere sowie deren Additionssalze mit einer pharmazeutisch annehmbaren Säure oder Base.

12. Verbindungen der Formel (I) nach Anspruch 1, worin X und X', die gleichartig oder verschieden sind, ein Sauerstoff- oder Schwefelatom bedeuten, deren Enantiomere, Diastereoisomere sowie deren Additionssalze mit einer pharmazeutisch annehmbaren Säure oder Base.

13. Verbindung der Formel (I) nach Anspruch 1, nämlich 7-Ethyl-2,3-methylendioxy-13-methyl-8,10-dioxo-8,9,10,12-tetrahydro-7*H*-cyclopenta[6,7]indolizino[1,2-b]chinolin-7-yl-3-piperidinopropanoat, dessen Enantiomere sowie dessen Additionssalze mit einer pharmazeutisch annehmbaren Säure oder Base.

14. Verbindung der Formel (I) nach Anspruch 1, nämlich 7-Ethyl-2,3-methylendioxy-13-cyclobutyl-8,10-dioxo-8,9,10,12-tetrahydro-7*H*-cyclopenta[6,7]-indolizino[1,2-b]chinolin-7-yl-3-piperidinopropanoat, dessen Enantiomere, Diastereoisomere sowie dessen Additionssalze mit einer pharmazeutisch annehmbaren Säure oder Base.

15. Verbindung der Formel (I) nach Anspruch 1, nämlich 7-Ethyl-2,3-methylendioxy-13-cyclobutyl-8,10-dioxo-8,9,10,12-tetrahydro-7*H*-cyclopenta[6,7]-indolizino[1,2-*b*]chinolin-7-yl-3-hexahydrocyclopenta[*c*]pyrrol-2(1*H*)-ylpropanoat, dessen Enantiomere sowie dessen Additionssalze mit einer pharmazeutisch annehmbaren Säure oder Base.

16. Verfahren zur Herstellung der Verbindungen der Formel (I) nach Anspruch 1, **dadurch gekennzeichnet, dass** man als Ausgangsprodukt eine Verbindung der Formel (II) verwendet, die wie in EP 1101765 beschrieben synthetisiert worden ist: 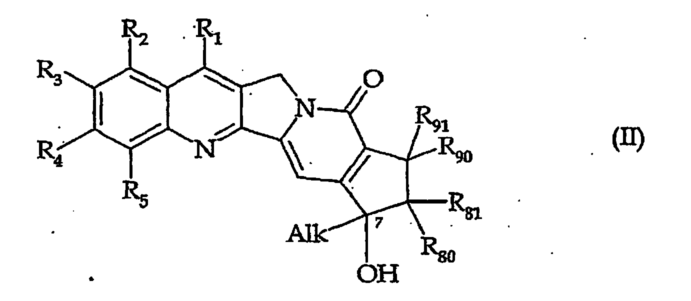 in der Alk, R₁, R₂, R₃, R₄, R₅, R₈₀, R₈₁, R₉₀ und R₉₁ die bezüglich der Formel (I) angegebenen Bedeutungen besitzen,
deren Hydroxygruppe in der C₇-Position in X"H umgewandelt wird, worin X" eine SH-, Amino- oder Alkylaminogruppe bedeutet, zur Bildung der Verbindung der Formel (III) 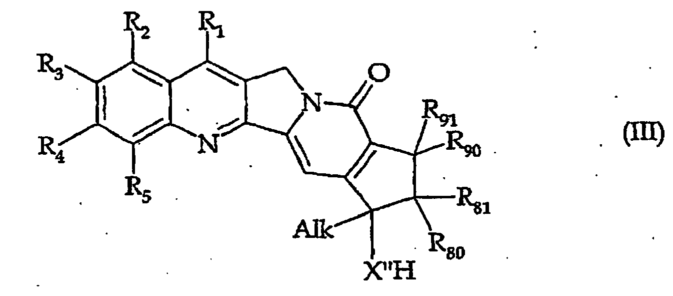 in der Alk, R₁, R₂, R₃, R₄, R₅, R₈₀, R₈₁, R₉₀ und R₉₁ die bezüglich der Formel (I) angegebenen Bedeutungen besitzen und X" die oben angegebenen Bedeutungen besitzt,
welche Verbindungen der Formel (II) oder (III) mit dem Reagens (IV): 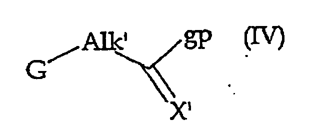 in der G, Alk' und X' die bezüglich der Formel (I) angegebenen Bedeutungen besitzen und gp eine austretende Gruppe bedeutet, wie Hal, OH, SH, NR'R" oder OC(O)R', worin R' und R" Alkylgruppen bedeuten,
kondensiert wird zur Bildung der Verbindung der Formel (I),
wobei es sich versteht, dass zur Vereinfachung des oben angegebenen Verfahrens die in R₈₀, R₈₁, R₉₀ und R₉₁ vorhandenen reaktiven Gruppen mit Hilfe von klassischen Schutzgruppen geschützt werden können und zu einem geeigneten Zeitpunkt von den Schutzgruppen befreit werden können, dass zu irgendeinem geeigneten Zeitpunkt der Synthese die in den gleichen Positionen vorhandenen Hydroxygruppen mit Hilfe klassischer Methoden der Chemie zu Oxogruppen oxidiert werden können und umgekehrt die in diesen gleichen Positionen vorhandenen Oxogruppen mit Hilfe klassischer Reduktionsmittel reduziert werden können und dass dann, wenn zwei dieser Gruppen gemeinsam eine Bindung bilden, diese zu irgendeinem von dem Fachmann als nützlich geeigneten Zeitpunkt eingeführt werden kann, um die Synthese zu erleichtern,
welche Verbindungen der Formel (I):
- gegebenenfalls mit Hilfe einer klassischen Reinigungsmethode gereinigt werden können,
- man gegebenenfalls mit Hilfe einer klassischen Trennungsmethode in die Stereoisomeren auftrennen kann und
- man gewünschtenfalls in ihre Additionssalze mit Hilfe einer pharmazeutisch annehmbaren Säure oder Base überführt.

17. Verbindungen der Formel (III'): 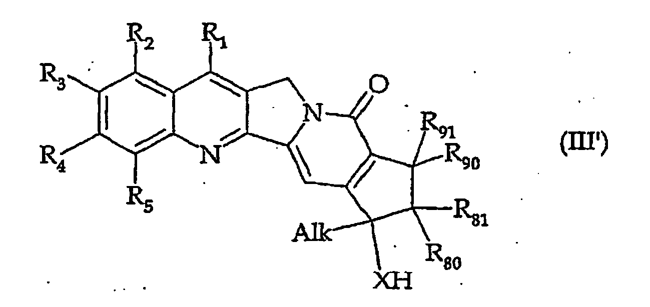 in der:
• Alk eine Alkylgruppe bedeutet,
• R₁, R₂, R₃, R₄ und R₅ unabhängig voneinander ausgewählt sind aus Wasserstoffatomen oder Halogenatomen, Alkyl-, Alkenyl-, Alkinyl-, Polyhalogenalkyl-, gegebenenfalls substituierten Cycloalkyl-, gegebenenfalls substituierten Cycloalkylalkyl-, Hydroxy-, Hydroxyalkyl-, Alkoxy-, Alkocyalkyl-, Nitro-, Cyano-, Acyloxy-gruppen, Gruppen -C(O)-R und Gruppen -(CH₂)ₚ₋NRₐR_{b} und -O-C(O)-N-RₐR_{b}, worin R eine Alkyl-, Alkoxy- oder (gegebenenfalls am Stickstoffatom durch eine oder zwei Alkylgruppen substituierte) Aminogruppe bedeutet, p eine ganze Zahl mit einem Wert zwischen 0 und 6 darstellt und Rₐ und R_{b} unabhängig voneinander ein Wasserstoffatom, eine Alkyl-, Cycloalkyl-, Cycloalkylalkyl-, Acyl-, gegebenenfalls substituierte Aryl- oder gegebenenfalls substituierte Arylalkylgruppe bedeuten oder Rₐ und R_{b} gemeinsam mit dem sie tragenden Stickstoffatom eine Pyrrolyl-, Piperidinyl- oder Piperazinylgruppe bedeuten, wobei jede dieser cyclischen Gruppen gegebenenfalls substituiert sein kann,
oder zwei benachbarte Gruppen R₂, R₃, R₄ und R₅ gemeinsam mit den sie tragenden Kohlenstoffatomen eine Gruppe -T-(CR_{c}R_{d})ₜT'- bilden, worin T und T', die gleichartig oder verschieden sind, ein Sauerstoffatom, ein Schwefelatom oder eine Gruppe N-Rₑ darstellen; R_{c} und R_{d}, die gleichartig oder verschieden sind, ein Wasserstoffatom oder ein Halogenatom bedeuten; t eine ganze Zahl mit einem Wert zwischen 1 und 3 einschließlich bedeutet; und Re ein Wasserstoffatom, eine Alkylgruppe oder eine Benzylgruppe bedeutet, mit der Maßgabe, dass mindestens eine der beiden Gruppen R_{c} oder R_{d} ein Halogenatom bedeutet, wenn T und T' jeweils ein Sauerstoffatom darstellen und X ein Sauerstoffatom bedeutet,
• R₈₀ und R₉₀ unabhängig voneinander ein Wasserstoffatom, eine Hydroxy-, Alkyl- oder Alkoxygruppe bedeuten,
• R₈₁ und R₉₁ unabhängig voneinander ein Wasserstoffatom, eine Alkyl-, Alkenyl- oder Alkinylgruppe bedeuten oder jeweils zwei Gruppen an benachbarten Kohlenstoffatomen gemeinsam eine Bindung bilden oder eine Oxirangruppe oder zwei geminale Gruppen (R₈₀ und R₈₁) und/oder (R₉₀ und R₉₁) gemeinsam eine Oxogruppe oder eine Gruppe -O-(CH₂)ₜ₁-O- bilden, wobei t₁ eine ganze Zahl mit einem Wert zwischen 1 und 3 einschließlich bedeutet,
• X ein Sauerstoff- oder Schwefelatom, eine Amino- oder Alkylaminogruppe bedeutet,
deren Enantiomere, Diastereoisomere sowie deren Additionssalze mit einer pharmazeutisch annehmbaren Säure oder Base.

18. Pharmazeutische Zubereitungen, enthaltend als Wirkstoff mindestens eine Verbindung nach einem der Ansprüche 1 bis 15, allein oder in Kombination mit einem oder mehreren inerten, nichttoxischen, pharmazeutisch annehmbaren Hilfsstoffen oder Trägermaterialien.

19. Pharmazeutische Zubereitungen nach Anspruch 18, enthaltend mindestens einen Wirkstoff nach einem der Ansprüche 1 bis 15, die für die Behandlung von Krebserkrankungen nützlich sind.
